# EUROPÄISCHE PATENTSCHRIFT

(11) **EP 3 662 990 B1**
(45) Veröffentlichungstag und Bekanntmachung des Hinweises auf die Patenterteilung: **07.07.2021**
(21) Anmeldenummer: 19200750.8
(22) Anmeldetag: 01.10.2019
(51) Int. Cl.: B01F 13/00, B01F 15/02

(54) **VORRICHTUNG ZUM MISCHEN EINES KNOCHENZEMENTS MIT HOHLRAUM ZUM MONOMERTRANSFER UND VERFAHREN**
DEVICE FOR MIXING A BONE CEMENT WITH A CAVITY FOR MONOMER TRANSFER AND PROCESS
DISPOSITIF DE MÉLANGE D'UN CIMENT OSSEUX POURVU DE CAVITÉ POUR UN TRANSFERT AU MONOMÈRE ET PROCÉDÉ

(30) Priorität: 07.12.2018 DE 102018131266
(43) Veröffentlichungstag der Anmeldung: 10.06.2020
(73) Patentinhaber: Heraeus Medical GmbH, 61273 Wehrheim (DE)
(72) Erfinder: Vogt, Sebastian Dr., 61273 Wehrheim (DE); Kluge, Thomas Dr., 61273 Wehrheim (DE)
(74) Vertreter: Schultheiss & Sterzel Patentanwälte PartG mbB

(56) Entgegenhaltungen:
- WO-A1-2012/115022
- JP-A- 2011 067 265
- US-A1- 2014 254 303

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs, mit einer solchen Vorrichtung.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs. Es handelt sich bei der erfindungsgemäßen Vorrichtung bevorzugt um ein Full-Prepacked-Mischsystem. Besonders bevorzugt ist die Vorrichtung so ausgebildet, dass ein Austragen von gemischtem Knochenzementteig ohne die Verwendung einer separaten Auspressvorrichtung möglich ist.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Konventionelle Polymethylmethacrylat-Knochenzemente (PMMA-Knochenzemente) sind aus einer pulverförmigen Komponente und einer flüssigen Monomerkomponente zusammengesetzt (K.-D. Kühn: Knochenzemente für die Endoprothetik: Ein aktueller Vergleich der physikalischen und chemischen Eigenschaften handelsüblicher PMMA-Zemente. Springer-Verlag Berlin Heidelberg New York, 2001). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt. PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2, der EP 0 796 653 A2, der US 5 588 745 A, der US 2018/333 176 A1, der US 2018/310 974 A1, der US 2018/289 406 A1, der US 2018/132 919 A1, der US 2018/132 917 A1 und der US 2018/256 233 A1 vorgeschlagen.

Das Patent DE 10 2009 031 178 B3 offenbart eine Lager- und Mischvorrichtung als Full-Prepacked-Mischsystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschließen einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt.

Diese einfachen mechanischen Auspressvorrichtungen nutzen insbesondere Klemmstangen zum Auspressen, die durch einen manuell zu betätigenden Kipphebel angetrieben werden. Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung vieler bisher bekannten Full-Prepacked-Mischsysteme muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Mit der JP 2011-067265 A wird eine Spritze zur Herstellung eines Knochenzements offenbart, die eine innenliegende innere Spritze und eine äußere Spritze aufweist. Dieses Dokument weist eine Vorrichtung auf mit einigen Merkmalen des Anspruchs 1 und ein Verfahren mit einigen Merkmalen des Anspruchs 12.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

In der US 2018/132 917 A1 und der US 2018/132 919 A1 wurden Full-Prepacked-Mischsysteme mit einer Kartusche enthaltend ein Knochenzementpulver vorgeschlagen. In der Kartusche ist ein Austragskolben vorgesehen und hinter der Kartusche eine Aufnahme enthaltend einen Monomerflüssigkeitsbehälter angeordnet. An der Rückseite der Aufnahme befindet sich ein Förderkolben, mit dem der Monomerflüssigkeitsbehälter zerquetscht werden kann und die Monomerflüssigkeit aus der Aufnahme in die Kartusche gepresst werden kann. Bei diesem Mischsystem wird Monomerflüssigkeit in verdichtetes Zementpulver eingepresst, wobei das Zementpulver durch die Monomerflüssigkeit benetzt wird und die zwischen den Zementpulverpartikeln befindliche Luft durch die Monomerflüssigkeit herausgepresst wird. Das bedeutet, es entsteht ein blasenfreier Zementteig ohne Einwirkung von mechanischen Mischvorrichtungen. Für die Funktion des Mischsystems ist es zwingend notwendig, eine separate mechanische Auspressvorrichtung mit dem Kartuschensystem zu verbinden. Durch manuelle Betätigung der Auspressvorrichtung wird zuerst der Monomerflüssigkeitsbehälter geöffnet, anschließend wird die Monomerflüssigkeit in das Zementpulver gepresst, wobei der Zementteig entsteht. Anschließend wird durch weitere Betätigung der Auspressvorrichtung der gebildete Zementteig aus der Kartusche ausgepresst. Üblich sind gegenwärtig resterilisierbare Auspressvorrichtungen, die nach Gebrauch gereinigt und sterilisiert werden müssen.

Die Aufgabe der vorliegenden Erfindung besteht darin, die Nachteile des Stands der Technik zu überwinden. Insbesondere besteht die Aufgabe der Erfindung in der Entwicklung einer Vorrichtung die zum Mischen des Knochenzementteigs aus den Ausgangskomponenten und zum Austragen des gemischten Knochenzementteigs vorgesehen und geeignet ist sowie eines Verfahrens zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer solchen Vorrichtung hergestellt wird, mit der die Nachteile der bisherigen Vorrichtungen und Verfahren überwunden werden. Die Erfindung hat die Aufgabe eine Vorrichtung wie die nach der US 2018/132 917 A1 und der US 2018/132 919 A1 derart zu verbessern, dass eine vorherbestimmbare Menge der Monomerflüssigkeit möglichst ohne Gaseinschlüsse oder Lufteinschlüsse in das Zementpulver eingepresst werden kann. Mit der erfindungsgemäßen Vorrichtung und dem erfindungsgemäßen Verfahren soll also erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und appliziert werden kann.

Die Vorrichtung soll möglichst ohne eine Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und gegebenenfalls auch das Öffnen des Monomerflüssigkeitsbehälters und gegebenenfalls auch das Öffnen der Kartusche mit möglichst wenigen Arbeitsschritten und so weit als möglich automatisiert ablaufen und vorzugsweise mit einem einzigen linearen Antrieb anzutreiben sein.

Die Aufgabe der Erfindung besteht also auch in der Entwicklung einer Vorrichtung zum Vermischen von Zementpulver und Monomerflüssigkeit. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung direkt betätigen können. Montage- und Arbeitsschritte sollen durch den Aufbau der Vorrichtung vermieden werden. In der Vorrichtung soll Zementpulver und Monomerflüssigkeit separat gelagert werden können. Bei der Vorrichtung soll es sich bevorzugt um ein Full-Prepacked-Mischsystem handeln. Eine Vermischung der beiden Zementkomponenten soll hierzu innerhalb weniger Sekunden in der geschlossenen Vorrichtung möglich sein, ohne dass ein manuelles Mischen mit Mischrädern beziehungsweise Mischflügeln notwendig ist. Die Vermischung soll dabei möglichst so erfolgen, dass der medizinische Anwender keine Berührung mit dem Zementpulver und der Monomerflüssigkeit hat. Das Mischsystem soll weiterhin so beschaffen sein, dass keine Montageschritte und auch kein externes Vakuum zum Monomertransfer notwendig sind. Das zu entwickelnde Mischsystem soll es ermöglichen, dass nach der Vermischung der beiden Zementkomponenten der gebildete Zementteig ohne Anschluss einer externen Auspressvorrichtung vorzugsweise durch manuelle Betätigung der Vorrichtung selbst ausgepresst werden kann. Die Vorrichtung soll eine Zubereitung und Applikation von Polymethylmethacrylat-Knochenzementteig ermöglichen, ohne dass Zusatzausrüstungen, wie Vakuumquellen, Vakuumschläuche und Auspressvorrichtungen erforderlich sind. Weiterhin soll der Transfer der Monomerflüssigkeit in der Weise erfolgen, dass nur Monomerflüssigkeit ohne Gaseinschlüsse beziehungsweise Luftblasen und nicht ein Gemisch aus Luft und Monomerflüssigkeit in das Zementpulver transferiert wird. Dadurch soll eine Bildung von Lufteinschlüssen im Knochenzementteig vermieden werden.

Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des Knochenzementteigs, die Vorrichtung aufweisend
A) eine Kartusche mit einem zylindrischen Innenraum,
B) einen Kartuschenkopf mit einer Austragsöffnung zum Austreiben des Knochenzementteigs, wobei der Kartuschenkopf die Kartusche an einer Vorderseite der Kartusche bis auf die Austragsöffnung verschließt,
C) einen Förderkolben, der im Innenraum der Kartusche angeordnet ist und der im Innenraum der Kartusche in Richtung der Austragsöffnung drückbar gelagert ist,
D) einen Austragskolben, der im Innenraum der Kartusche zwischen der Austragsöffnung und dem Förderkolben angeordnet ist und der im Innenraum der Kartusche in Richtung der Austragsöffnung drückbar gelagert ist, wobei eine dem Förderkolben zugewandte Rückseite des Austragskolbens eine sich in Richtung des Kartuschenkopfs verjüngende Vertiefung aufweist,
E) einen ersten Hohlraum, der von dem Kartuschenkopf, von Innwänden der Kartusche und von dem Austragskolben begrenzt ist, wobei das Zementpulver in dem ersten Hohlraum angeordnet ist,
F) einen zweiten Hohlraum, der ein Teil des zylindrischen Innenraums der Kartusche ist, wobei der zweite Hohlraum von dem Austragskolben und dem Förderkolben begrenzt ist,
G) einen rückseitigen Teil des Innenraums der Kartusche, dessen Vorderseite von einer von dem Austragskolben abgewandten Rückseite des Förderkolbens begrenzt ist,
H) ein Leitungsmittel, das den zweiten Hohlraum mit dem rückseitigen Teil des Innenraums der Kartusche an dem Förderkolben vorbei und für die Monomerflüssigkeit durchlässig verbindet, wobei das Leitungsmittel durch ein Verschieben des Förderkolbens in Richtung des Austragskolbens von dem Förderkolben gegen den zweiten Hohlraum verschließbar ist, und
I) eine Durchführung, wobei die Durchführung an einer Spitze der sich verjüngenden Vertiefung beginnt, sich durch den Austragskolben erstreckt und den ersten Hohlraum und den zweiten Hohlraum für die Monomerflüssigkeit durchlässig aber für das Zementpulver undurchlässig miteinander verbindet.

Es kann bevorzugt vorgesehen sein, dass das Leitungsmittel zumindest eine Nut oder zumindest eine Bypass-Leitung ist. Die zumindest eine Nut kann bevorzugt in der Innenwand der Kartusche angeordnet sein. Die zumindest eine Nut kann sich ebenfalls bevorzugt in axialer Richtung des Innenraums entlang eines Teils des Innenraums erstrecken. Durch die zumindest eine Nut oder die zumindest eine Bypass-Leitung kann die Monomerflüssigkeit in den zweiten Hohlraum am Förderkolben vorbei einleitbar sein.

Aufgrund der leichteren Fertigung wird die zumindest eine Nut gegenüber zumindest einer Bypass-Leitung oder anderer Leitungsmittel als Leitungsmittel erfindungsgemäß bevorzugt.

Es kann vorgesehen sein, dass das Leitungsmittel, insbesondere die zumindest eine Nut oder die zumindest eine Bypass-Leitung, in einem Bereich in Richtung der Rückseite der Kartusche angeordnet ist.

Es kann vorgesehen sein, dass sich das Leitungsmittel, insbesondere die zumindest eine Nut, in axialer Richtung des Innenraums entlang eines Teils des Innenraums erstreckt.

Dass sich das Leitungsmittel, insbesondere die zumindest eine Nut, in axialer Richtung des Innenraums entlang eines Teils des Innenraums erstreckt, bedeutet nicht zwangsläufig, dass sich das Leitungsmittel, insbesondere die zumindest eine Nut, parallel zur axialen Richtung des Innenraums erstreckt, sondern dass sie sich nicht senkrecht zur axialen Richtung des Innenraums erstreckt, es sei denn eine entsprechende Nut wäre ausreichend breit, um einen Monomerflüssigkeitstransfer in den zweiten Hohlraum am Förderkolben vorbei zu ermöglichen. Das Leitungsmittel, insbesondere die zumindest eine Nut, kann dabei linear oder gekrümmt sein. Es wird jedoch erfindungsgemäß besonders bevorzugt, wenn das Leitungsmittel, insbesondere die zumindest eine Nut, sich parallel zur axialen Richtung des Innenraums erstreckt, da die Kartusche mit dem Leitungsmittel, insbesondere der zumindest einen Nut, dann etwas einfacher zu konstruieren und zu fertigen ist.

Eine einzelne ringförmige aber ausreichend breite Nut hat den Nachteil, dass der Förderkolben beim Überfahren derselben keinen Halt hat, wenn keine Führungsstreben vorhanden sind, die die Nut überbrücken und die den Förderkolben an seinem radialen Umfang beim Überfahren der Nut führen.

Es kann bevorzugt auch vorgesehen sein, dass als die zumindest eine Nut mehrere Nuten oder eine Vielzahl von Nuten verwendet werden. Der konstruktive Aufwand hierfür ist nicht sehr hoch und der Monomerflüssigkeitstransfer wird durch den größeren Gesamtleitungsquerschnitt verbessert.

Insbesondere kann das Leitungsmittel durch ein Verschieben des Förderkolbens in Richtung des Austragskolbens von dem Förderkolben gegen den zweiten Hohlraum flüssigkeitsdicht verschließbar sein.

Dass das Leitungsmittel den zweiten Hohlraum mit dem rückseitigen Teil des Innenraums der Kartusche an dem Förderkolben vorbei verbindet, bedeutet vorzugsweise, dass die axiale Erstreckung vom Anfang bis zum Ende des Leitungsmittel einen größeren Abstand hat, als die axiale Erstreckung des an dem Innenraum der Kartusche anliegenden Teils des Förderkolbens, insbesondere als die axiale Erstreckung des Teils des Förderkolbens, der zum Verschließen des Leitungsmittels vorgesehen ist.

Es sei an diese Stelle ausdrücklich darauf hingewiesen, dass der Knochenzementteig aus der Vorrichtung auf einen Spatel oder in ein Gefäß zur späteren Verwendung ausgetragen werden kann. Eine direkte Applikation an einem Patienten ist also nicht notwendig und auch nicht beansprucht.

Während der Förderkolben in Richtung des Austragskolbens gedrückt wird und dabei das Leitungsmittel, insbesondere die zumindest eine Nut oder die zumindest eine Bypass-Leitung, überfährt und dabei verschließt, kann sich der zweite Hohlraum fortwährend verkleinern.

Bevorzugt ist die erfindungsgemäße Vorrichtung auch zum Lagern des Zementpulvers vorgesehen und besonders bevorzugt auch zum Lagern der Monomerflüssigkeit vorgesehen.

Bevorzugt kann vorgesehen sein, dass an der Vorderseite der Kartusche, insbesondere am Kartuschenkopf, ein Austragsrohr befestigbar ist, wobei besonders bevorzugt das Austragsrohr die Austragsöffnung begrenzt.

Es kann vorgesehen sein, dass der Innenraum der Kartusche durch eine Innenwand der Kartusche begrenzt ist.

Der Förderkolben kann an seiner dem Austragskolben gegenüberliegenden Seite bombiert sein.

Bevorzugt kann vorgesehen sein, dass das Volumen des zweiten Hohlraums mindestens gleich dem Volumen der verwendeten Monomerflüssigkeit ist, insbesondere dem Volumen der Monomerflüssigkeit in einem Monomerflüssigkeitsbehälter der Vorrichtung ist. Bevorzugt ist das Volumen des zweiten Hohlraums mindestens um ein Drittel größer als das Volumen der verwendeten Monomerflüssigkeit, insbesondere das Volumen der Monomerflüssigkeit in einem Monomerflüssigkeitsbehälter der Vorrichtung.

Der Innenraum der Kartusche hat bis auf die von dem Leitungsmittel verursachten Asymmetrien, insbesondere bis auf die zumindest eine Nut oder die Einmündungen zu der zumindest einen Bypass-Leitung eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum bevorzugt, da diese am einfachsten zu fertigen ist.

Es kann vorgesehen sein, dass eine dem Austragskolben zugewandte Vorderseite des Förderkolbens eine sich in Richtung des Austragskolbens vorspringende Oberfläche aufweist, die sich vorzugsweise in Richtung des Austragskolbens stetig verjüngt.

Hiermit wird erreicht, dass Gas- oder Lufteinschlüsse gut durch das Leitungsmittel, insbesondere durch die zumindest eine Nut, entweichen können, wenn die Vorrichtung beim Einleiten der Monomerflüssigkeit aus dem rückseitigen Teil des Innenraums der Kartusche in den zweiten Hohlraum mit dem Kartuschenkopf nach unten gehalten wird.

Es kann vorgesehen sein, dass die sich in Richtung des Kartuschenkopfs verjüngende Vertiefung eine sich von der Innenwand der Kartusche aus stetig verjüngende Vertiefung in Form einer trichterförmigen Oberfläche ist, wobei bevorzugt eine vorspringende Oberfläche an der Vorderseite des Förderkolbens in die trichterförmige Oberfläche an der Rückseite des Austragskolbens einschiebbar ist, wobei besonders bevorzugt die Durchführung im Austragskolben an der tiefsten Stelle der trichterförmigen Oberfläche in den Austragskolben mündet.

Hierdurch kann sichergestellt werden, dass sich eine zwischen dem Förderkolben und dem Austragskolben befindliche Monomerflüssigkeit zu einem großen Anteil von dem zweiten Hohlraum in den ersten Hohlraum drücken lässt und dass Lufteinschlüsse oder Gaseinschlüsse sich leicht aus dem zweiten Hohlraum in den ersten Hohlraum überführen, insbesondere eindrücken lassen.

Besonders bevorzugt ist die trichterförmige Vertiefung an der Rückseite des Austragskolbens eine Negativform der vorspringenden Oberfläche an der Vorderseite des Förderkolbens.

Gemäß einer bevorzugten Weiterbildung der vorliegenden Erfindung kann vorgesehen sein, dass eine dem Austragskolben zugewandte Vorderseite des Förderkolbens und die dem Förderkolben zugewandte Rückseite des Austragskolbens zueinander flächenbündig passende Formen aufweisen, so dass die Vorderseite des Förderkolbens an der Rückseite des Austragskolbens flächenbündig anliegt, wenn der Förderkolben gegen den Austragskolben gedrückt ist, wobei vorzugsweise das Volumen des zweiten Hohlraums dadurch auf maximal 1% des Volumens des zweiten Hohlraums in einem Ausgangszustand reduzierbar ist.

Hierdurch wird erreicht, dass eine in den zweiten Hohlraum eingeleitete Monomerflüssigkeit vollständig oder zu einem großen Anteil in den ersten Hohlraum gepresst werden kann, indem der Förderkolben bei bereits verschlossenem Leitungsmittel in Richtung des Austragskolbens gedrückt wird.

Des Weiteren kann vorgesehen sein, dass die dem Förderkolben zugewandte Rückseite des Austragskolbens eine trichterförmiger Oberfläche als die sich verjüngende Vertiefung aufweist, in deren tiefsten Punkt die Durchführung angeordnet ist, und eine dem Austragskolben zugewandte Vorderseite des Förderkolbens eine vorspringende kegelförmige Oberfläche mit der gleichen Steigung wie die trichterförmige Oberfläche an der Rückseite des Austragskolbens aufweist, wobei vorzugsweise an der Spitze der vorspringenden kegelförmigen Oberfläche an der Vorderseite des Austragskolbens ein zylindrischer Stift angeordnet ist, der in eine zylindrische Vertiefung als Teil der Durchführung im Austragskolben einsteckbar ist.

Auf diese Weise kann zum einen das Zurückhalten von Gas- oder Lufteinschlüssen im zweiten Hohlraum verhindert werden und zum anderen können beim Einfüllen der Monomerflüssigkeit in den zweiten Hohlraum Gas- oder Lufteinschlüsse leicht durch die Durchführung entweichen. Durch Austreiben von Gaseinschlüssen beziehungsweise Lufteinschlüssen zwischen den Pulverpartikeln des Zementpulvers hindurch kann ein blasenfreier oder blasenarmer Knochenzementteig hergestellt werden. Ferner kann dadurch die in dem zweiten Hohlraum enthaltene Monomerflüssigkeit vollständig oder größtenteils in den ersten Hohlraum transferiert werden.

Dabei kann bevorzugt vorgesehen sein, dass die trichterförmige Oberfläche an der Rückseite des Austragskolbens und die vorspringende kegelförmige Oberfläche an der Vorderseite des Förderkolbens den gleichen Fußdurchmesser haben.

Ferner kann vorgesehen sein, dass die trichterförmige Oberfläche an der Rückseite des Austragskolbens einen Steigungswinkel von mindestens 45° aufweist, bevorzugt von mindestens 55°, besonders bevorzugt von mindestens 60°, und die kegelförmige Oberfläche an der Vorderseite des Förderkolbens einen dazu passenden Steigungswinkel von mindestens 45° aufweist, bevorzugt von mindestens 55°, besonders bevorzugt von mindestens 60°aufweist.

Hiermit wird bei senkrechter Aufstellung der Vorrichtung mit dem Kartuschenkopf nach oben ein schnelles Entweichen von Gaseinschlüssen aus dem zweiten Hohlraum in den ersten Hohlraum ermöglicht.

Es kann vorgesehen sein, dass der Austragskolben und/oder der Förderkolben gegen eine Innenwand der Kartusche abgedichtet ist oder sind, wobei die Innenwand der Kartusche den Innenraum der Kartusche begrenzt. Dabei kann bevorzugt vorgesehen sein, dass der Austragskolben und/oder der Förderkolben mit zumindest einem Dichtungsring gegen die Innenwand der Kartusche abgedichtet ist oder sind. Hierbei kann wiederum besonders bevorzugt vorgesehen sein, dass der zumindest eine Dichtungsring in zumindest einer umlaufenden Nut im Austragskolben und/oder der Förderkolben angeordnet ist oder sind.

Durch die Abdichtung wird verhindert, dass die Monomerflüssigkeit an dem Austragskolben und/oder dem Förderkolben vorbei gedrückt werden kann und so einen negativen Einfluss auf das gewünschte Mischungsverhältnis haben kann oder eine Kontamination der Umgebung verursacht.

Es kann gemäß einer bevorzugten Ausführung der vorliegenden Erfindung vorgesehen sein, dass an der Rückseite des Förderkolbens ein Behälter angeordnet ist, in dem ein Monomerflüssigkeitsbehälter enthaltend die Monomerflüssigkeit angeordnet ist, insbesondere eine Ampulle aus Glas oder Kunststoff, wobei der Monomerflüssigkeitsbehälter innerhalb des Behälters zu öffnen ist und wobei der Behälter über zumindest eine Monomerleitung mit dem rückseitigen Teil des Innenraums der Kartusche flüssigkeitsdurchlässig verbunden ist, wobei bevorzugt ein Öffnungsmittel an einer dem Förderkolben gegenüberliegende Seite des Behälters angeordnet ist, mit dem der Monomerflüssigkeitsbehälter innerhalb des Behälters zu öffnen ist, wobei besonders bevorzugt das Öffnungsmittel eine an einer Kappe befestigte Hülse ist, wobei die Kappe auf ein Gewinde des Behälters schraubbar ist und die Kappe hierzu ein Gegengewinde aufweist, so dass beim Aufschrauben der Kappe der Monomerflüssigkeitsbehälter, insbesondere die Ampulle, mit der Hülse auf zumindest einen vorstehenden Stift an der Innenseite des Behälters gedrückt wird und dadurch der Monomerflüssigkeitsbehälter, insbesondere die Ampulle, aufbrechbar ist.

Hiermit wird ein Full-Prepacked-Mischsystem bereitgestellt, in dem alle Ausgangskomponenten des Knochenzementteigs, nämlich die Monomerflüssigkeit und das Zementpulver, in der Vorrichtung gelagert und gemischt werden können. Das Hantieren mit der Monomerflüssigkeit kann so innerhalb der Vorrichtung erfolgen und der Anwender ist vor dem Zementpulver und insbesondere vor der Monomerflüssigkeit geschützt.

Es kann bevorzugt vorgesehen sein, dass der Behälter an dem Förderkolben befestigt ist.

Hierdurch kann eine gemeinsame Bewegung des Förderkolbens mit dem Behälter synchronisiert werden. Zudem kann so eine stabilere Bewegung des Förderkolbens erreicht werden. Der Förderkolben kann dabei axial drehbar gegen den Behälter gelagert sein, wobei die Drehachse auf der Achse des zylindrischen Innenraums der Kartusche liegt. Hierdurch kann sich der Förderkolben bei einem Einschrauben des Behälters linear und ohne Drehung im Innenraum der Kartusche bewegen.

Es kann ein lösbares Sicherungselement vorgesehen, das eine Bedienung des Öffnungsmittels verhindert.

Bevorzugt steckt der Monomerflüssigkeitsbehälter in einer Passung in dem Behälter, so dass der Monomerflüssigkeitsbehälter in dem Behälter gehalten wird. An der Einmündung zu der zumindest einen Monomerleitung kann in dem Behälter ein Sieb oder ein Filter zum Zurückhalten von Splittern des Monomerflüssigkeitsbehälters angeordnet sein.

Die Hülse kann ein Rohrstück sein. Die Hülse kann auf Schultern einer Ampulle als Monomerflüssigkeitsbehälter drücken, so dass die Ampulle in dem Behälter an einem Ampullenboden der Ampulle aufgedrückt wird. Bevorzugt ist die Hülse einteilig mit der Kappe oder bildet mit der Kappe eine Einheit.

Als Monomerflüssigkeitsbehälter können vorzugsweise Glasampullen, Kunststoffampullen, Plastikampullen, Kunststoffbeutel, Folienbeutel, Kunststoffverbundbeutel und Aluminium-Kunststoffverbundbeuteln verwendet werden, die zur Lagerung der Monomerflüssigkeit geeignet sind.

Ferner kann bevorzugt auch vorgesehen sein, dass in der Wandung des Behälters oder im Öffnungsmittel zumindest eine Belüftungsöffnung angeordnet ist, die den Innenraum des Behälters mit der Umgebung verbindet.

Hierdurch kann der Innenraum des Behälters auf einfach Weise evakuiert und mit einem sterilisierenden Gas sterilisiert werden.

Gemäß einer bevorzugten Weiterbildung der vorliegenden Erfindung kann auch vorgesehen sein, dass die zumindest eine Belüftungsöffnung derart dicht im Bereich einer Schraubkappe, insbesondere einer Schraubkappe als Öffnungsmittel zum Öffnen eines Monomerflüssigkeitsbehälters, in dem Behälter angeordnet ist, so dass die zumindest eine Belüftungsöffnung durch eine Bewegung der Schraubkappe in Richtung der Kartusche verschlossen wird, bevor ein in dem Behälter angeordneter Monomerflüssigkeitsbehälter, in dem die Monomerflüssigkeit enthalten ist, durch die Bewegung der Schraubkappe geöffnet ist.

Hierdurch kann die Monomerflüssigkeit nicht aus dem Innenraum des Behälters austreten, wenn die zumindest eine Belüftungsöffnung von der sich in Richtung der Kartusche bewegenden Schraubkappe verschlossen wird, bevor der Monomerflüssigkeitsbehälter durch die Bewegung der Schraubkappe geöffnet wird, also beispielsweise im Innenraum des Behälters zerdrückt, zersplittert, aufgestochen oder aufgerissen wird.

Der Behälter kann einen zylindrischen Innenraum aufweisen, der zur Aufnahme einer Ampulle als Monomerflüssigkeitsbehälter mit zylindrischen Außenumfang geeignet ist. Der Behälter kann dann als Ampullenhalter bezeichnet werden, insbesondere dann, wenn der Innendurchmesser des Innenraums des Behälters zum Außendurchmesser der einzusetzenden Ampulle passt.

Im Innenraum des Behälters kann ein Vorsprung zum Aufbrechen, Aufstechen oder Aufschneiden des Monomerflüssigkeitsbehälters angeordnet sein, wobei der Vorsprung insbesondere ein Stechdorn oder Anstechdorn ist. Der Vorsprung ist dabei vorzugsweise in einer Einmündung des Innenraums des Behälters zu der zumindest einen Monomerleitung.

Bei erfindungsgemäßen Vorrichtungen mit Behälter kann auch vorgesehen sein, dass der Behälter ein Außengewinde aufweist, das in ein Innengewinde an einem dem Kartuschenkopf gegenüberliegenden Ende der Kartusche schraubbar ist, wobei durch Einschrauben des Behälters in die Kartusche der Förderkolben in Richtung der Austragsöffnung drückbar ist und der Austragskolben mit dem Förderkolben in Richtung der Austragsöffnung drückbar ist, wobei bevorzugt das Innengewinde Teil einer Ringhülse ist, die an dem dem Kartuschenkopf gegenüberliegenden Ende der Kartusche mit der Kartusche verbunden ist.

Hierdurch kann die Vorrichtung durch eine Schraubbewegung von außen bedient werden. Die Schraubbewegung hat den Vorteil, dass ein kraftvoller Antrieb des Förderkolbens und des Austragskolbens ermöglicht wird, so dass auch zähe Knochenzementteige mit der Vorrichtung aus der Kartusche ausgepresst werden können.

Ferner kann vorgesehen sein, dass die Außenseite des Behälters in einem ersten Abschnitt ausgehend von einer dem Kartuschenkopf zugewandten Stirnseite kein Außengewinde besitzt und in einem zweiten Abschnitt ein Außengewinde besitzt.

Hierdurch kann das Leitungsmittel durch Vorschieben des Behälters in den Innenraum der Kartusche und damit des Förderkolbens zunächst verschlossen werden, bevor das Außengewinde des Förderkolbens in das Innengewinde der Kartusche greift. Dadurch, dass die beiden Schritte zeitlich voneinander getrennt werden, kann eine Fehlbedienung der Vorrichtung auf einfache Weise verhindert werden.

Des Weiteren kann vorgesehen sein, dass das Leitungsmittel an einer Stelle an der Innenwand der Kartusche endet oder einmündet, die von dem Austragskolben derart axial bezogen auf die Achse des zylindrischen Innenraums derart beabstandet ist, dass das Volumen des zweiten Hohlraums mindestens so groß ist wie das Volumen der durch das Leitungsmittel eingeleiteten Monomerflüssigkeit, insbesondere der in dem Monomerflüssigkeitsbehälter enthaltenen Monomerflüssigkeit, wenn der Förderkolben gerade so weit in Richtung des Austragskolbens verschoben ist, dass der Förderkolben das Leitungsmittel gegen der zweiten Hohlraum verschließt.

Hierdurch wird ermöglicht, dass die Monomerflüssigkeit vollständig in den zweiten Hohlraum einleitbar ist und dass Gase aus dem zweiten Hohlraum austreten können, so dass nur die Monomerflüssigkeit in dem zweiten Hohlraum enthalten ist, wenn das Verschlussmittel das Leitungsmittel verschließt.

Auch kann vorgesehen sein, dass ein Stopfen in der Austragsöffnung angeordnet ist, der die Austragsöffnung für das Zementpulver undurchlässig verschließt, insbesondere für Gase durchlässig verschließt, wobei vorzugsweise der Stopfen in der Austragsöffnung beweglich angeordnet ist, so dass der Stopfen durch einen Druck auf den fertig gemischten Knochenzementteig aus der Austragsöffnung heraus gedrückt werden kann, wobei besonders bevorzugt ein von außen sichtbares Markierungsmittel an dem Stopfen befestigt ist, an dessen Position von außen ablesbar ist, ob der Stopfen in der Austragsöffnung nach außen gedrückt ist.

Hiermit kann ein vorzeitiges Austreten des Knochenzementteigs verhindert werden. Durch die bevorzugte Ausgestaltung wird erreicht, dass von außen erkennbar ist, wenn der Knochenzementteig bis zur Austragsöffnung fertig gemischt ist. Dies ergibt daraus, dass der Knochenzementteig fließfähig ist, während das Zementpulver nicht fließfähig ist, so dass der Stopfen nur dann bewegt wird, wenn der Knochenzementteig fertig gemischt ist oder das Zementpulver zumindest vollständig benetzt ist und der Austragskolben über den Knochenzementteig ein Druck auf den Stopfen vermittelt.

Der Stopfen kann Teil eines Verschlusssystems sein, mit dem die Austragsöffnung verschlossen ist. Dabei kann der Stopfen in einer zylindrischen Bohrung des Verschlusssystems in axialer Richtung beweglich angeordnet sein. Das Verschlusssystem kann mit einem Außengewinde in ein Innengwinde an einem Stutzen des Kartuschenkopfs eingeschraubt sein. Selbstverständlich kann das Verschlusssystem alternativ auch eine Kappe mit einem Innengewinde aufweisen, das auf einen Stutzen am Kartuschenkopf mit einem Außengewinde geschraubt ist. Der Stutzen kann nach Entfernen des Verschlusssystems mit einem Austragsrohr verbunden werden.

Die Kartusche ist vorzugsweise hohlzylinderförmig. Der Kartuschenkopf kann konisch geformt sein. Dadurch kann ein Gas leichter aus dem Kartuschenkopf austreten. Zudem verbleibt ein Totvolumen innerhalb des Kartuschenkopfs, wenn der Austragskolben bis zum Kartuschenkopf gedrückt ist. An dieser Stelle können weniger gut gemischte Anteile des Knochenzementteigs zurückgehalten werden, die nicht ausgetragen werden können.

Die Durchführung ist vorzugsweise an der Spitze oder im Kegelmantel einer kegelförmigen Vertiefung an der Rückseite des Austragskolbens angeordnet.

Es kann auch vorgesehen sein, dass das Volumen der verwendeten Monomerflüssigkeit, insbesondere das Volumen der in dem Monomerflüssigkeitsbehälter enthaltenen Monomerflüssigkeit, zumindest höchstens so groß ist, wie das Volumen des zweiten Hohlraums.

Gemäß einer bevorzugten Ausführung der vorliegenden Erfindung kann vorgesehen sein, dass das Zementpulver so in dem ersten Hohlraum verdichtet ist, dass die Zementpulverpartikel nicht frei beweglich sind. Hierdurch wird sichergestellt, dass sich die Monomerflüssigkeit aufgrund von Kapillarkräften schnell und gleichmäßig in dem Zementpulver ausbreiten kann.

Es kann vorgesehen sein, dass ein lösbares Sicherungsmittel mit dem Förderkolben verbunden ist, das eine Bewegung des Förderkolbens gegen die Kartusche verhindert. Bevorzugt ist das Sicherungsmittel an dem Behälter für den Monomerflüssigkeitsbehälter angeordnet und blockiert ein Einschieben oder Einschrauben des Behälters in die Kartusche und damit eine Bewegung des Förderkolbens in der Kartusche.

Die Kartusche ist vorzugsweise aus einem thermoplastischen Kunststoff gefertigt, insbesondere mit einem Spritzgussverfahren. Besonders bevorzugt sind auch die anderen Teile der Vorrichtung, wie der Austragskolben, der Förderkolben und der Kartuschenkopf, sowie gegebenenfalls der Behälter und der Stopfen aus einem thermoplastischen Kunststoff gefertigt, insbesondere mit einem Spritzgussverfahren.

Es kann ferner vorgesehen sein, dass die Durchführung durch ein für Gase und Flüssigkeiten undurchlässigen Stopfen verschlossen ist, wobei der Stopfen in der Durchführung in Richtung Kartuschenkopf verschiebbar ist, wobei bevorzugt an der Vorderseite des Förderkolbens ein Stift angeordnet ist, mit dem der Stopfen in der Durchführung aus der Durchführung herausdrückbar ist und damit die Durchführung zu öffnen ist, wobei besonders bevorzugt, der Stift eine solche axiale Erstreckung bezüglich des zylindrischen Innenraums hat, dass bei einer Berührung des Stifts mit dem Stopfen, das Volumen zwischen der Rückseite des Austragskolbens und der Vorderseite des Förderkolbens im Innenraum der Kartusche zumindest genauso groß ist, wie das Volumen der Monomerflüssigkeit in einem Monomerflüssigkeitsbehälter, der in der Vorrichtung angeordnet ist oder anzuordnen ist.

Hierdurch wird erreicht, dass die Monomerflüssigkeit nicht vorzeitig in den ersten Hohlraum eindringen kann, damit sie dort nicht mit dem Zementpulver quillt und dort eine gelartige Barriere bildet, die einer weiteren Ausbereitung der Monomerflüssigkeit in dem Zementpulver entgegenwirken kann.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer vorher beschriebenen erfindungsgemäßen Vorrichtung hergestellt wird, gekennzeichnet durch die folgenden nacheinander ablaufenden Schritte:
A) Einleiten der Monomerflüssigkeit durch das Leitungsmittel in den zweiten Hohlraum,
B) Drücken des Förderkolbens in Richtung des Austragskolbens, bis der Förderkolben alle Verbindungen des Leitungsmittels zum zweiten Hohlraum verschließt,
C) Halten der Vorrichtung mit dem Kartuschenkopf nach oben und weiteres Drücken des Förderkolbens in Richtung des Austragskolbens, wobei Luft oder Gas aus dem ersten Hohlraum und aus dem zweiten Hohlraum durch den Kartuschenkopf entfernt wird und wobei die Monomerflüssigkeit aus dem zweiten Hohlraum in das Zementpulver in den ersten Hohlraum gedrückt wird,
D) Drücken des Austragskolbens mit dem Förderkolben in Richtung der Austragsöffnung, wobei der in dem ersten Hohlraum gebildete Knochenzementteig durch die Austragsöffnung ausfließt.

Bevorzugt kann vorgesehen sein, dass bei dem erfindungsgemäßen Verfahren keine Behandlung eines menschlichen oder tierischen Körpers erfolgt, insbesondere keine von der Patentierung ausgeschlossene Behandlung eines menschlichen oder tierischen Körpers erfolgt.

Bevorzug kann ferner vorgesehen sein, dass sich der Knochenzementteig während Schritt C) und/oder nach Schritt C) aber vor Schritt D) in dem ersten Hohlraum bildet.

Es kann vorgesehen sein, dass die Vorrichtung in Schritt A), und vorzugsweise auch in Schritt B), mit dem Kartuschenkopf nach unten aufgestellt oder gehalten wird und in Schritt C) Gaseinschlüsse aus dem zweiten Hohlraum durch das die Durchführung, den ersten Hohlraum und die Austragsöffnung im Kartuschenkopf entweichen, vorzugsweise durch einen für Gase durchlässigen aber für das Zementpulver undurchlässigen Filter in der Austragsöffnung entweichen, wobei der Filter die Austragsöffnung für Gase durchlässig und für das Zementpulver undurchlässig verschließt..

Hierdurch kann eine genau vorbestimmte Menge der Monomerflüssigkeit in das Zementpulver eingetragen werden. Zudem kann so ein blasenfreier oder blasenarmer Knochenzementteig erzeugt werden.

Ferner kann vorgesehen sein, dass in Schritt D) der Förderkolben flächenbündig an dem Austragskolben anliegt und bevorzugt in Schritt C) das Volumen des zweiten Hohlraums vollständig oder bis auf maximal 1% reduziert wird.

Dadurch kann die Monomerflüssigkeit vollständig aus dem zweiten Hohlraum in das Zementpulver in dem zweiten Hohlraum gepresst werden.

Des Weiteren kann vorgesehen sein, dass in Schritt D) durch den auf den Knochenzementteig wirkenden Druck ein Stopfen in der Austragsöffnung bewegt oder hervorgedrückt wird, wobei bevorzugt hierauf der Stopfen aus der Austragsöffnung entfernt wird und besonders bevorzugt danach ein Applikationsrohr an dem Kartuschenkopf der Kartusche befestigt wird.

Hierdurch kann ein Anwender der Vorrichtung erkennen, wann der Knochenzementteig einsatzbereit ist.

Es kann auch vorgesehen sein, dass vor Schritt A) ein Monomerflüssigkeitsbehälter enthaltend die Monomerflüssigkeit in einem Behälter geöffnet wird und die Monomerflüssigkeit in dem Behälter freigesetzt wird, wobei der Behälter an einer dem Austragskolben abgewandten Rückseite des Förderkolbens angeordnet ist, insbesondere befestigt ist, und die Monomerflüssigkeit in Schritt A) aus dem Behälter durch das Leitungsmittel in den zweiten Hohlraum fließt, wobei vorzugsweise in Schritt B) und C) der Förderkolben und in Schritt D) der Förderkolben und der Austragskolben durch Eindrücken oder Einschrauben des Behälters in die Kartusche angetrieben werden.

Hierdurch kann das Verfahren auf einfache Weise auch manuell durchgeführt werden. Die Vorrichtung kann als Full-Prepacked-Mischsystem verwendet werden und ein Kontakt des Anwenders mit der Monomerflüssigkeit kann während des Verfahrens ausgeschlossen werden.

Der Erfindung liegt die überraschende Erkenntnis zugrunde, dass es durch ein von einem Förderkolben verschließbares Leitungsmittel in einer Wandung einer Kartusche gelingt, einen Hohlraum (vorliegend den zweiten Hohlraum) nach außen oder gegen einen hinteren Teil eines Innenraums der Kartusche durch eine Bewegung des Förderkolbens in den Innenraum der Kartusche hinein zu verschließen und dadurch ein nach außen geschlossenes aber in Richtung eines (ersten) Hohlraums mit dem Zementpulver darin für die Monomerflüssigkeit durchlässig verbundenes Monomerflüssigkeitsreservoir bereitzustellen, das durch eine weitere Bewegung des Förderkolbens in die Kartusche hinein in das Zementpulver drückbar ist, wobei dabei der zweite Hohlraum immer weiter verkleinert wird und wobei das Monomerflüssigkeitsreservoir bei geeigneter Orientierung der Vorrichtung frei oder im Wesentlichen frei von Gas- beziehungsweise Lufteinschlüssen ist. Um die in dem zweiten Hohlraum enthaltene Monomerflüssigkeit in das Zementpulver zu drücken und um den fertigen Knochenzementteig auszutreiben, reicht es aus, den Förderkolben in Richtung der Austragsöffnung zu drücken. Dabei verschließt sich zunächst das Leitungsmittel. Anschließend wird die Monomerflüssigkeit in den ersten Hohlraum mit dem Zementpulver transferiert und danach kann der gebildete Knochenzementteig durch gemeinsames Vortreiben des Förderkolbens mit dem Austragskolben aus der Kartusche ausgetrieben werden. Durch die in der Rückseite des Austragskolbens vorgesehene sich verjüngende Vertiefung wird erreicht, dass sich Gase oder Luft an der Durchführung sammeln können, wenn die Vorrichtung mit dem Kartuschenkopf nach oben gehalten wird, so dass das Gas oder die Luft durch die Durchführung in den ersten Hohlraum gedrückt werden kann und von dort durch die nachfließende Monomerflüssigkeit nach oben zur Austragsöffnung steigt und durch die Austragsöffnung herausgedrückt werden kann. Auf diese Weise kann ein blasenarmer oder blasenfreier Knochenzementteig erzeugt werden. Durch die geringere Dichte des Gases gegenüber der Monomerflüssigkeit steigt das Gas in der sich zur Durchführung hin verjüngenden Vertiefung auf und die sich verjüngende Vertiefung wirkt aufgrund der Ausrichtung der Vorrichtung als Separator für die Monomerflüssigkeit und einem eingeschlossenen Gas. Aufgrund der sich verjüngenden Form kann sich keine Gastasche bilden und keine Bläschen werden zurückgehalten. Insbesondere bei einer Kegelform ist auch keine exakt senkrecht Haltung der Vorrichtung mit dem Kartuschenkopf nach oben notwendig, solange nur die Durchführung an der höchsten Stelle der kegelförmigen beziehungsweise trichterförmigen sich verjüngenden Vertiefung angeordnet ist und sich keine anderen Gewölbe bilden, in denen Gasblasen zurückgehalten werden könnten.

Bevorzugt kann zuvor die Monomerflüssigkeit aus einem an die Rückseite des Förderkolbens angeschlossenen und mit der Fluidöffnung verbundenen Behälter durch das Leitungsmittel in den zweiten Hohlraum geleitet werden. Der Förderkolben kann dabei durch Einschieben und/oder Einschrauben des Behälters in die Kartusche im Innenraum der Kartusche in Richtung der Austragsöffnung gedrückt werden. Besonders bevorzugt kann zuvor ein Monomerflüssigkeitsbehälter innerhalb des Behälters geöffnet werden, um die Monomerflüssigkeit innerhalb des Behälters freizusetzen. Diese besonders bevorzugte erfindungsgemäße Vorrichtung hat die wesentlichen Vorteile, dass die beiden Ausgangskomponenten des Knochenzementteigs im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Es handelt sich dann um ein Full-Prepacked-Mischsystem. Der medizinische Anwender hat keinerlei Kontakt mit den einzelnen Ausgangskomponenten des Knochenzementteigs. Die Geruchsbelästigung ist dadurch nur noch minimal.

Ein besonderer Vorteil der Vorrichtung besteht auch darin, dass durch das einfache Vorwärtsbewegen des Förderkolbens bei geschlossenem Leitungsmittel die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Voraussetzung dafür ist die Verwendung eines Zementpulvers, das so eingestellt ist, dass es sehr gut von der Monomerflüssigkeit benetzt wird und diese durch Kapillarwirkung einsaugen kann. Die Bedienung der Vorrichtung ist maximal vereinfacht.

Die Vorteile erfindungsgemäßer Vorrichtungen und Verfahren beruhen grundsätzlich darauf, dass die an sich bekannte lineare Bewegungen so genutzt werden, um den Monomertransfer durchzuführen und um den zweiten Hohlraum nach außen zu verschließen.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Eine beispielhafte und bevorzugte erfindungsgemäße Vorrichtung zum Lagern, Vermischen und Austragen von Polymethylmethacrylat-Knochenzement kann aufweisen:
a) eine hohlzylinderförmige Kartusche,
b) einen Kartuschenkopf, der die Kartusche verschließt ist, wobei der Kartuschenkopf eine Auslassöffnung aufweist, die den Innenraum der Kartusche mit der Umgebung verbindet,
c) einen für Gase durchlässigen und für Pulverpartikel undurchlässigen Stopfen als Kartuschenverschluss, der lösbar in der Auslassöffnung des Kartuschenkopfs angeordnet ist,
d) einen Austragskolben, der im Innenraum der Kartusche axial beweglich angeordnet ist, wobei zumindest ein Teil der Rückseite des Austragskolbens als Hohlkegel ausgebildet ist, der in eine Öffnung mündet, die für Pulverpartikel undurchlässig und für Gase und Flüssigkeiten durchlässig ist, und die Vorderseite und die Rückseite des Austragskolbens gas-und flüssigkeitsdurchlässig verbindet,
e) Zementpulver, das in einem ersten Hohlraum der Kartusche angeordnet ist, der vom Kartuschenkopf mit dem Kartuschenverschluss, der Innenwand der Kartusche und dem Austragskolben begrenzt ist,
f) einen für Flüssigkeiten und Gase undurchlässigen Förderkolben, der hinter dem Austragskolben im Innenraum der Kartusche angeordnet ist, wobei der Förderkolben an der dem Austragskolben abgewandten Stirnseite bombiert ist, und wobei die Vorderseite des Förderkolbens als Kegel ausgebildet ist, wobei der Kegel den gleichen Fußdurchmesser und die gleiche Steigung wie der Hohlkegel des Austragskolbens hat,
g) einen zweiten Hohlraum, der durch den Austragskolben, die Kartuscheninnenwand und den Förderkolben begrenzt ist,
h) mindestens eine axial an der Innenseite der Kartusche verlaufenden Nut, die eine axiale Erstreckung hat, die länger ist als die axiale Erstreckung des Förderkolbens, wobei die Nut zu mindestens teilweise in den zweiten Hohlraum ragt,
i) einen hohlzylinderförmigen Ampullenhalter als Behälter, der zumindest teilweise im Innenraum der Kartusche angeordnet ist, wobei die Außenseite des Ampullenhalters in einem ersten Abschnitt ausgehend von einer dem Kartuschenkopf zugewandten Stirnseite kein Außengewinde besitzt und in einem zweiten Abschnitt ein Außengewinde besitzt, wobei vorzugsweise der Ampullenhalter an seiner dem Kartuschenkopf zugewandten Stirnseite mit einer Siebplatte und einer für Flüssigkeit und Gase durchlässigen und für Pulverpartikel undurchlässigen Porenscheibe verschlossen ist und wobei die auf der zur Innenraum des Ampullenhalters zeigenden Stirnseite der Siebplatte mindestens einen Anstechdorn besitzt,
j) ein Öffnungsmittel am Ampullenhalter, das eine Kappe mit einem Innengewinde aufweist, und wobei das Innengewinde des Öffnungsmittels in das Außengewinde des Ampullenhalters greift,
k) eine im Ampullenhalter axial verschiebbaren Hülse, die zwischen einem Monomerflüssigkeitsbehälter in dem Ampullenhalter oder dem Öffnungsmittel angeordnet ist und die mit dem Öffnungsmittel gegen den Monomerflüssigkeitsbehälter axial verschiebbar ist,
l) ein Innengewinde an einem dem Kartuschenkopf gegenüberliegenden Ende der Kartusche, wobei
m) der Förderkolben durch axiale Bewegung des Ampullenhalters in Richtung des Kartuschenkopfs so über die mindestens eine axiale Nut verschiebbar ist, dass der zweite Hohlraum in Richtung des Ampullenhalters flüssigkeitsundurchlässig verschlossen ist.

Bevorzugt ist der hohlzylinderförmige Ampullenhalter axial in der Kartusche beweglich, wobei der Ampullenhalter an seiner dem Kartuschenkopf zugewandten Stirnseite als Förderkolben in der Form eines Kegels ausgebildet ist.

Die erfindungsgemäße Vorrichtung ist vorteilhaft vom Anwender zu nutzen, weil der Anwender nur den Verschlusskopf des Ampullenhalters einschrauben und die Vorrichtung mit dem Kartuschenkopf nach oben drehen muss und nach wenigen Sekunden einen fertig gemischten Knochenzementteig erhält, der keine oder praktisch keine Lufteinschlüsse enthält. Der Prozess des Öffnens des Monomerflüssigkeitsbehälters, des Monomertransfers und der Vermischung laufen geordnet nacheinander nur durch einfache wiederholte Drehung des Verschlusskopfs des Ampullenhalters mit dem verbundenen Ampullenhalter und durch eine geeignete Ausrichtung der Vorrichtung automatisch ab. Es sind dadurch die aus der bisherigen Zementiertechnik bekannten Anwenderfehler beim Öffnen der Monomerflüssigkeitsbehälter, dem Monomertransfer und dem Vermischen der Monomerflüssigkeit mit dem Zementpulver konstruktiv ausgeschlossen. Dadurch wird die Anwendersicherheit deutlich erhöht. Die Vorrichtung ist dabei auch ohne weiteres von ungelernten Hilfskräften einsetzbar.

Ein erfindungsgemäßes Verfahren kann beispielsweise mit der beispielhaften Vorrichtung zur Vermischung des Zementpulvers mit der Monomerflüssigkeit unter Bildung von Knochenzementteig umgesetzt werden. Ein beispielhaftes und erfindungsgemäß bevorzugtes Verfahren kann mit den folgenden und nacheinander ablaufenden Schritten umgesetzt werden:
Positionieren oder Halten der Kartusche mit dem Kartuschenkopf nach unten,
Drehen des Verschlusskopfs auf das Außengewinde des Ampullenhalters,
Verschieben der Hülse gegen den Monomerflüssigkeitsbehälter,
Bewegen des Monomerflüssigkeitsbehälters gegen den Stechdorn,
Öffnen des Monomerflüssigkeitsbehälters,
Ausfließen der Monomerflüssigkeit aus dem Monomerflüssigkeitsbehälter durch die zumindest eine Nut in den zweiten Hohlraum und bevorzugt gegebenenfalls zuvor auch durch die Siebplatte und die Porenscheibe entlang der Bombierung des Förderkolbens,
Verschieben des Ampullenhalters in Richtung des Kartuschenkopfs, bis das Außengewinde des Ampullenhalters in das Innengewinde der Kartusche greift, wobei gleichzeitig der Förderkolben so in Richtung des Kartuschenkopfs geschoben wird, dass der Förderkolben über die mindestens eine Nut geschoben und der zweite Hohlraum in Richtung des Ampullenhalters flüssigkeitsundurchlässig verschlossen wird,
Umdrehen der Vorrichtung, so dass der Kartuschenkopf nach oben zeigt,
Einschrauben des Ampullenhalters in die Kartusche, wobei das Außengewinde des Ampullenhalters sich in das Innengewinde der Kartusche dreht und der Ampullenhalter in Richtung Kartuschenkopf bewegt wird,
Bewegen des Förderkolbens in Richtung Kartuschenkopf,
Auspressen der Monomerflüssigkeit aus dem zweiten Hohlraum durch den Hohlkegel im Austragskolben in das verdichtete Zementpulver,
Benetzen des Zementpulvers mit der Monomerflüssigkeit,
Bildung des Knochenzementteigs durch Anquellen des mit Monomerflüssigkeit benetzten Zementpulvers,
Auspressen der gesamten Monomerflüssigkeit aus dem zweiten Hohlraum durch Einschieben des Kegels des Förderkolbens in den Hohlkegel des Austragskolbens,
Verdrängen der Luft zwischen den Zementpulverpartikeln durch die Monomerflüssigkeit, Entweichen der Luft durch den Kartuschenverschluss in die Umgebung,
Entfernen des Kartuschenverschlusses nach Bildung des Zementteigs,
Drehen des Ampullenhalters in Richtung des Kartuschenkopfs, und
bevorzugt Auspressen des Knochenzementteigs durch Bewegen des Ampullenhalters in Richtung des Kartuschenkopfs.

Weiterhin ist ein zweites Verfahren erfindungsgemäß bevorzugt. Dieses Verfahren zum Vermischen und Austragen von Polymethylmethacrylat-Knochenzement mit der beispielhaften erfindungsgenmäßen Vorrichtung ist durch folgende nacheinander ablaufende Schritte charakterisiert:
Positionieren oder Halten der Kartusche mit dem Kartuschenkopf nach unten,
Drehen des Verschlusskopfs auf das Außengewinde des Ampullenhalters,
Verschieben der Hülse gegen den Monomerflüssigkeitsbehälter,
Bewegen des Monomerflüssigkeitsbehälters gegen den Stechdorn,
Öffnen des Monomerflüssigkeitsbehälters,
Ausfließen der Monomerflüssigkeit aus dem Monomerflüssigkeitsbehälter durch die zumindest eine Nut in den zweiten Hohlraum und bevorzugt gegebenenfalls zuvor auch durch die Siebplatte und die Porenscheibe entlang der Bombierung des Förderkolbens,
Verschieben des Ampullenhalters in Richtung des Kartuschenkopfs, bis das Außengewinde des Ampullenhalters in das Innengewinde der Kartusche greift, wobei gleichzeitig der Förderkolben so in Richtung des Kartuschenkopfs geschoben wird, dass der Förderkolben über die mindestens eine Nut geschoben und der zweite Hohlraum in Richtung des Ampullenhalters flüssigkeitsundurchlässig verschlossen wird,
Umdrehen der Vorrichtung, so dass der Kartuschenkopf nach oben zeigt,
Bewegen des Förderkolbens in Richtung Kartuschenkopf,
Komprimieren der Luft oberhalb der Monomerflüssigkeit,
Herausdrücken des Verschlusses aus einer Öffnung im Austragskolben durch die komprimierte Luft und die komprimierte Monomerflüssigkeit oder durch einen Ausstoßer am Förderkolben,
Entweichen der Luft durch den Hohlkegel in die Öffnung des Austragskolbens und nachfolgendes Auspressen der Monomerflüssigkeit durch die Öffnung des Austragskolbens, Auspressen der Monomerflüssigkeit aus dem zweiten Hohlraum durch den Hohlkegel im Austragskolben in das verdichtete Zementpulver,
Benetzen des Zementpulvers mit der Monomerflüssigkeit,
Bildung des Knochenzementteigs durch Anquellen des mit Monomerflüssigkeit benetzten Zementpulvers,
Auspressen der gesamten Monomerflüssigkeit aus dem zweiten Hohlraum durch Einschieben des Kegels des Förderkolbens in den Hohlkegel des Austragskolbens,
Verdrängen der Luft zwischen den Zementpulverpartikeln durch die Monomerflüssigkeit,
Entweichen der Luft durch den Kartuschenverschluss in die Umgebung,
Entfernen des Kartuschenverschlusses nach Bildung des Zementteigs,
Drehen des Ampullenhalters in Richtung des Kartuschenkopfs, und
bevorzugt Auspressen des Knochenzementteigs durch Bewegen des Ampullenhalters in Richtung des Kartuschenkopfs.

Eine Variante beider beispielhafter Verfahren ist durch folgende nacheinander ablaufende Schritte charakterisiert, dass beim Verschieben des beweglichen Stopfens ein mit dem beweglichen Stopfen verbundener farbiger Stift als Markierungsmittel hervortritt und dadurch die erfolgte Vermischung des Zementpulvers mit der Monomerflüssigkeit dem medizinischen Anwender anzeigt.

Im Folgenden werden weitere Ausführungsbeispiele der Erfindung anhand von neun schematisch dargestellten Figuren erläutert, ohne jedoch dabei die Erfindung zu beschränken. Dabei zeigt:
Figur 1: eine schematische Querschnittansicht einer beispielhaften erfindungsgemäßen Vorrichtung zum Lagern und Mischen einer Monomerflüssigkeit und eines Zementpulvers in einem Ausgangs- beziehungsweise Lagerungszustand der Vorrichtung;
Figur 2: eine schematische perspektivische Querschnittansicht der Vorrichtung nach Figur 1 ohne die Monomerflüssigkeit und dass Zementpulver;
Figur 3: eine schematische perspektivische Außenansicht der Vorrichtung nach den Figuren 1 und 2;
Figur 4: eine schematische Querschnittansicht der Vorrichtung nach den Figuren 1 bis 3 mit einem geöffnetem Monomerflüssigkeitsbehälter darin;
Figur 5: eine schematische Querschnittansicht einer Ausschnittvergrößerung der Vorrichtung nach den Figuren 1 bis 4 kurz nach dem Verschließen des Leitungsmittels; und
Figur 6: eine schematische Querschnittansicht einer Ausschnittvergrößerung der Vorrichtung nach den Figuren 1 bis 5 während des Einpressens der Monomerflüssigkeit in das Zementpulver, wobei die Vorrichtung mit dem Kartuschenkopf nach oben gedreht wurde; und
Figur 7: eine schematische Querschnittansicht der Vorrichtung nach den Figuren 1 bis 6 mit vorgeschobenem Stopfen und Markierungsmittel als Anzeige zur Einsatzbereitschaft des Knochenzementteigs;
Figur 8: eine schematische Querschnittansicht der Vorrichtung nach den Figuren 1 bis 7 beim Austragen des Knochenzementteigs; und
Figur 9: eine schematische Querschnittansicht der Vorrichtung nach den Figuren 1 bis 8 nach dem Austragen des Knochenzementteigs.

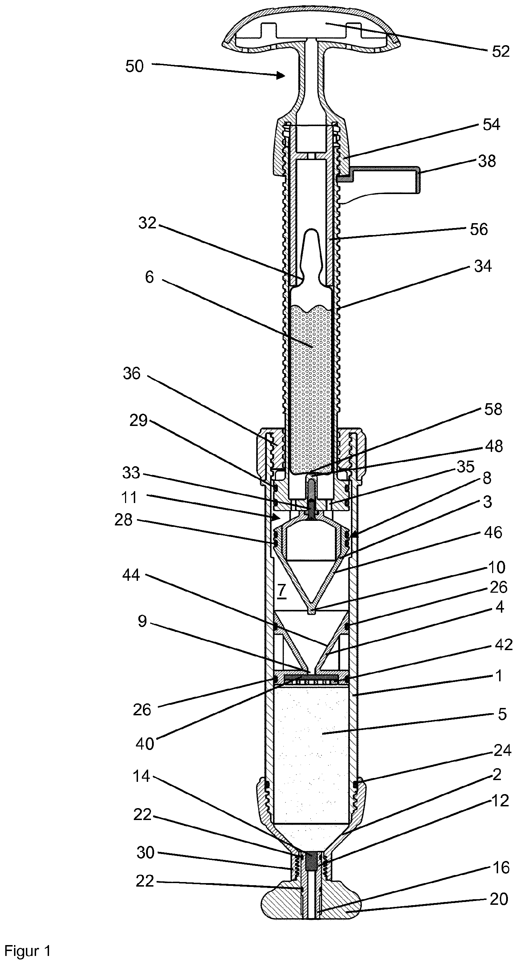

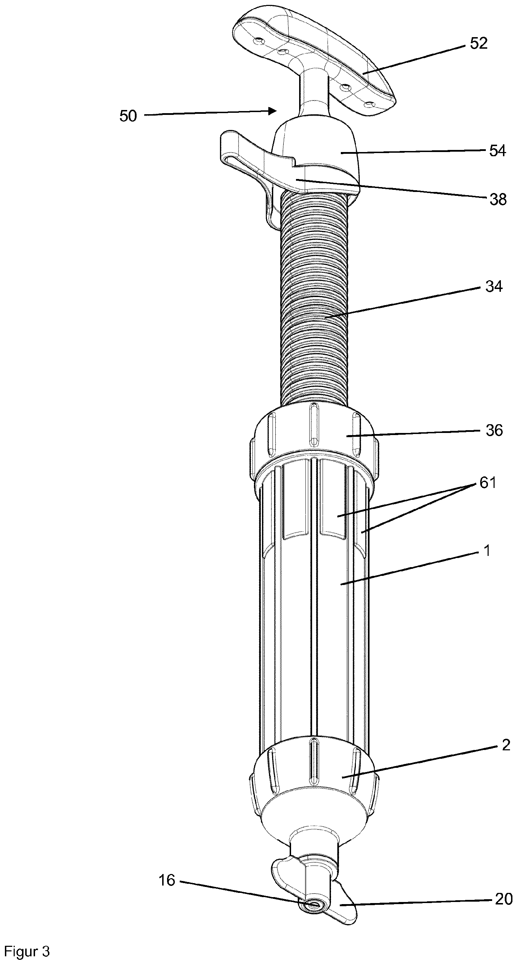

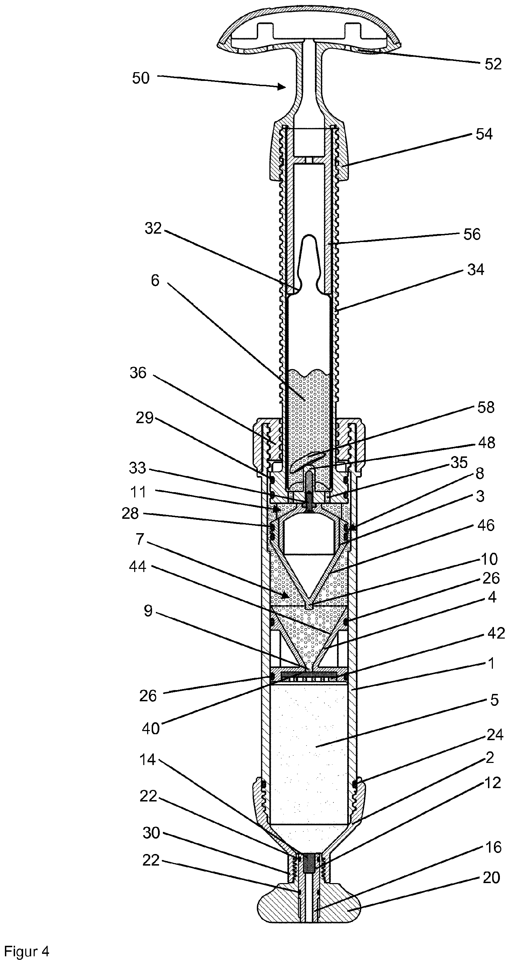

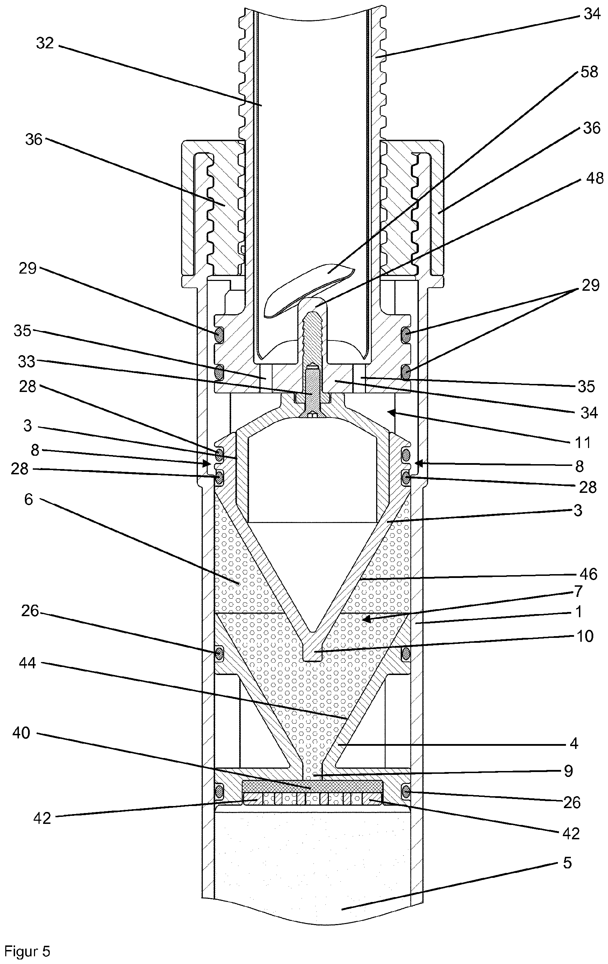

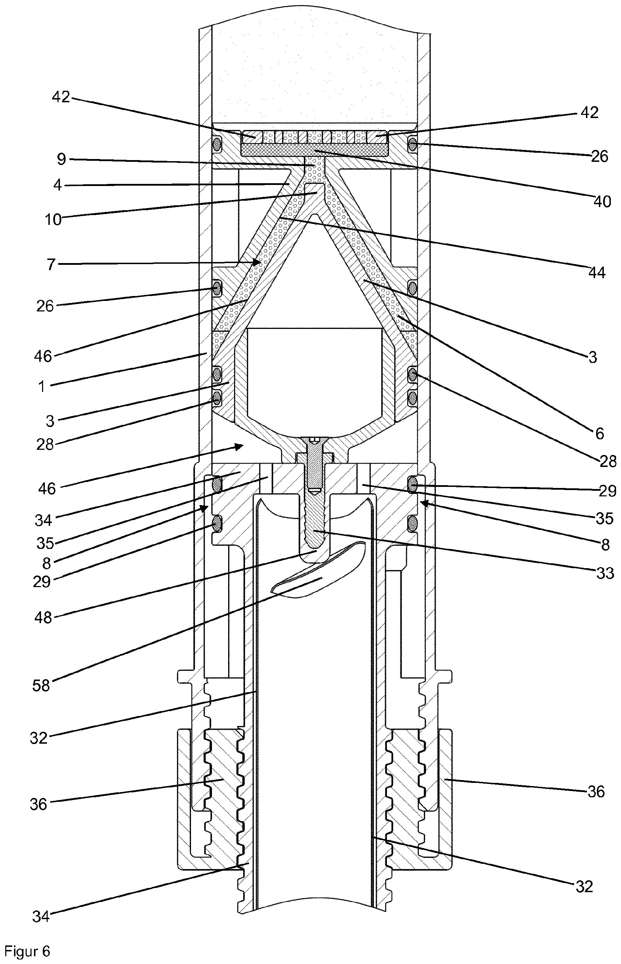

In den Figuren 1 bis 9 sind Abbildungen einer erfindungsgemäßen Vorrichtung gezeigt. Die Figuren 1 bis 4 und 7 bis 9 zeigen verschiedene schematische Gesamtansichten der beispielhaften erfindungsgemäßen Vorrichtung. Die Figuren 5 und 6 zeigen schematische Querschnittansichten als Detailansichten in Form von Ausschnittvergrößerungen durch einen Bereich der erfindungsgemäßen Vorrichtung. Die Figuren 1 bis 3 zeigen dabei die erste erfindungsgemäße Vorrichtung in einem Ausgangszustand oder Lagerungszustand, während die Figuren 4 bis 9 Querschnittansichten der ersten erfindungsgemäßen Vorrichtung während der Benutzung der Vorrichtung zur Verdeutlichung eines beispielhaften erfindungsgemäßen Verfahrens zeigen.

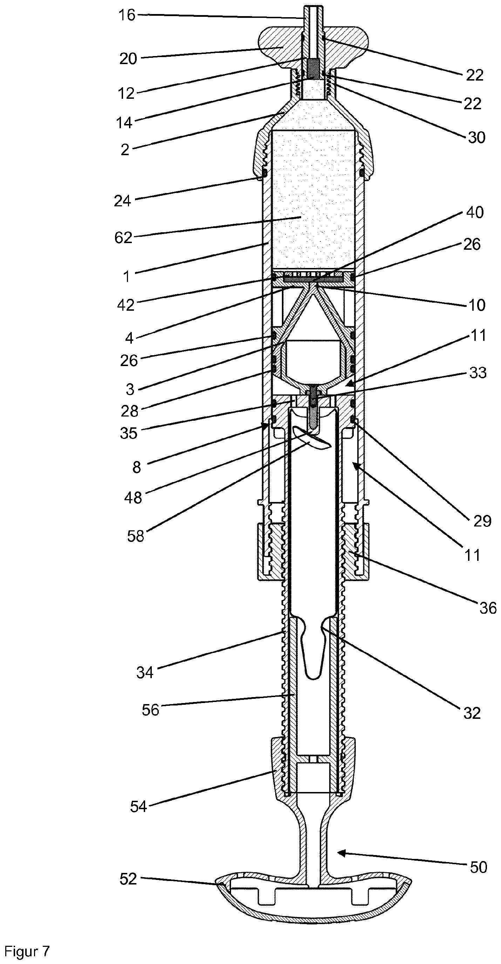

Gemäß einem Ausführungsbeispiel der vorliegenden Erfindung hat die Vorrichtung eine röhrenförmigen Kartusche 1 aus einem Kunststoff mit einem zylindrischen Innenraum. Die Kartusche 1 kann an ihrer Vorderseite mit einem trichterförmigen Kartuschenkopf 2 aus Kunststoff verschlossen sein. Die Vorderseite der Kartusche 1 ist in den Figuren 1 bis 5 und 8 und 9 unten und in den Figuren 6 und 7 oben, damit Luft oder Gas nach oben aus der Vorrichtung entweichen kann. An der Spitze des trichterförmigen Kartuschenkopfs 2 kann eine zentrale Austragsöffnung angeordnet sein, die im Ausgangszustand aber zunächst verschlossen sein kann. Der Kartuschenkopf 2 kann gemäß einer alternativen Ausführung aber auch eine flache Kappe sein oder eine andere Form aufweisen. Um Luft- oder Gaseinschlüsse im Bereich des Kartuschenkopfs zu vermeiden, wird jedoch ein trichterförmiger Kartuschenkopf 2 erfindungsgemäß bevorzugt. Es ist grundsätzlich auch möglich, den Kartuschenkopf 2 einteilig mit der Kartusche 1 auszuführen. Um die Montage der Vorrichtung zu erleichtern, wird jedoch bevorzugt, wenn der Kartuschenkopf 2 als separates Teil mit der Kartusche 1 verbunden wird, beispielsweise aufgeschraubt oder aufgesteckt wird.

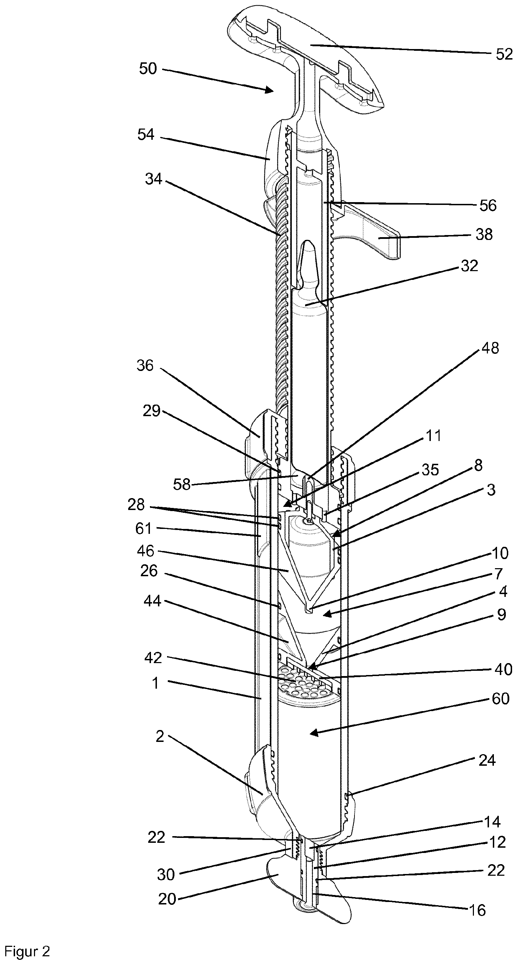

Gemäß einer bevorzugten Ausführung kann an einer der Vorderseite der Kartusche 1 gegenüberliegenden Rückseite der Kartusche 1 im Innenraum der Kartusche 1 ein Förderkolben 3 angeordnet sein, der axial im Innenraum der Kartusche 1 in Richtung der Vorderseite der Kartusche 1 beweglich gelagert ist. Zwischen dem Förderkolben 3 und der Vorderseite der Kartusche 1 kann ein Austragskolben 4 angeordnet sein. Der Förderkolben 3 und der Austragskolben 4 können zumindest teilweise aus Kunststoff gefertigt sein. Zwischen dem Austragskolben 4 und der Vorderseite der Kartusche 1 beziehungsweise dem Kartuschenkopf 2 ist ein erster Hohlraum 60 im Inneren der Vorrichtung gebildet (siehe Figur 2). In dem ersten Hohlraum 60 kann ein Zementpulver 5 als eine der Ausgangskomponenten des zu fertigenden Knochenzementteigs 62 (siehe Figuren 7 bis 9) enthalten sein. Bevorzugt ist das Zementpulver 5 zwischen dem Austragskolben 4 und dem Kartuschenkopf 2 im Kartuschenkopf 2 und in dem Innenraum der Kartusche 1 im ersten Hohlraum 60 eingepresst oder zumindest kompakt gelagert, um ein Einleiten und Verteilen einer Monomerflüssigkeit 6 in dem Zementpulver 5 durch Nutzung von Kapillarkräften zwischen den Zementpulverpartikeln zu vereinfachen.

Von dem Austragskolben 4 und dem Förderkolben 3 und den Innenwänden der Kartusche 1 kann im Innenraum der Kartusche 1 ein zweiter Hohlraum 7 begrenzt sein. Mehrere Nuten als für die Monomerflüssigkeit 6 durchlässige Leitungsmittel 8 können an der Innenwand der Kartusche 1 angeordnet sein, durch die die Monomerflüssigkeit 6 in den zweiten Hohlraum 7 eingeleitet werden kann. Die Nuten können sich beziehungsweise das Leitungsmittel 8 kann sich in axialer Richtung des Innenraums der Kartusche 1 erstrecken. Die Nuten erstrecken sich vorzugsweise nicht bis in den ersten Hohlraum 60. Die Nuten können in radialer Richtung bei verschiedenen Winkeln voneinander beabstandet sein. In dem Austragskolben 4 kann eine Durchführung 9 vorgesehen sein, die an der Spitze einer trichterförmigen Vertiefung beginnt und durch die die Monomerflüssigkeit 6 aus dem zweiten Hohlraum 7 zu dem Zementpulver 5 in dem ersten Hohlraum 60 fließen oder geleitet werden kann (siehe Figur 6).

Der Austragskolben 4 kann an seiner Rückseite eine trichterförmige Oberfläche 44 aufweisen, die eine Negativform zu einer kegelförmigen Oberfläche 46 an der Vorderseite des Förderkolbens 3 bildet. Dabei kann bevorzugt vorgesehen sein, dass der Austragskolben 4 mit seiner trichterförmigen Oberfläche 44 flächenbündig an der kegelförmigen Oberfläche 46 des Förderkolbens 3 anliegen kann, wenn der Förderkolben 3 gegen den Austragskolben 4 gedrückt ist (siehe Figuren 7 bis 9). Das Volumen des zweiten Hohlraums 7 kann dabei auf null reduziert sein, so dass der zweiten Hohlraum 7 in diesem Zustand verschwunden ist.

Gemäß einer bevorzugten Ausführung kann vorgesehen sein, dass an der Spitze der kegelförmigen Oberfläche 46 an der Vorderseite des Förderkolbens 3 ein Stift 10 in Form eines zylindrischen Stifts angeordnet ist. Der Außenumfang des Stifts 10 kann dabei etwas kleiner als die Durchführung 9 sein, so dass die Durchführung 9 mit dem Stift 10 nicht flüssigkeitsdicht verschließt. Dazu kann der Außenumfang des Stifts 10 etwas kleiner als der Innenumfang der Durchführung 9 sein.

In der Durchführung 9 kann gemäß einer Weiterbildung des vorliegenden Ausführungsbeispiels ein Stopfen (nicht gezeigt) angeordnet sein, der die Durchführung 9 im Lagerungszustand beziehungsweise Ausgangszustand zunächst gasdicht und flüssigkeitsdicht verschließt. Dieser Stopfen in der Durchführung 9 kann mit dem Stift 10 aus der Durchführung 9 herausgedrückt werden, um die Durchführung 9 zum ersten Hohlraum 60 hin zu öffnen. Der Stift 10 kann dabei derart langestreckt sein, dass er ab Unterschreiten eines Mindestabstands zwischen dem Austragskolben 4 und dem Förderkolben 3 auf den Stopfen in der Durchführung 9 trifft, wobei das Leitungsmittel 8 in dieser Stellung des Förderkolbens 3 bereits durch den Förderkolben 3 gegenüber dem zweiten Hohlraum 7 verschlossen ist. Hierdurch ist der zweite Hohlraum 7 nach außen gegenüber einem rückseitigen Teil 11 des Innenraums der Kartusche 1 verschlossen, bevor die Durchführung 9 des zweiten Hohlraums 7 zum ersten Hohlraum 60 geöffnet wird. Der Stift 10 kann vorzugsweise durch die Durchführung 9 hindurch geschoben werden, wenn der Förderkolben 3 in Richtung des Austragskolbens 4 gedrückt wird.

Gemäß einer bevorzugten Ausführung kann die Durchführung 9 und der Stift 10 entlang oder parallel zur Zylinderachse des zylindrischen Innenraums der Kartusche 1 angeordnet sein und miteinander fluchten.

Für den verständigen Fachmann ist dabei klar, dass diese Ausführung mit einer zentralen Durchführung im Austragskolben 4 ohne weiteres auf eine nicht zentrale Durchführung übertragbar ist. Ebenso kann ein nicht auf der Zylinderachse des Innenraums der Kartusche 1 angeordneter Stift realisiert werden. Es können auch ohne weiteres mehrere Stifte an der Vorderseite des Förderkolbens 4 angeordnet sein.

Ferner können ohne statt vieler Nuten nur eine Nut als Leitungsmittel 8 zum Durchleiten der Monomerflüssigkeit 6 von dem rückseitigen Teil 11 des Innenraums der Kartusche 1 in den zweiten Hohlraum 7 vorgesehen sein. Ebenso können das Leitungsmittel 8 auch durch zumindest eine flüssigkeitsdurchlässige Leitung realisiert werden, die im Bereich des rückseitigen Teils 11 des Innenraums der Kartusche 1 beginnt und die vor dem Förderkolben 3 im Ausgangszustand (Figur 1) in zweiten Hohlraum 7 mündet, wobei die Einmündung in den zweiten Hohlraum 7 von dem Förderkolben 3 überfahrbar und damit gegenüber dem zweiten Hohlraum 7 flüssigkeitsdicht verschließbar ist. Das Leitungsmittel 8 kann dann durch Schläuche oder Bohrungen in der Wandung der Kartusche 1 realisiert werden. Ein für die Realisierung der vorliegenden Erfindung wichtiger und zentraler Aspekt ist darin zu sehen, dass das alle Verbindungen des Leitungsmittels 8 zum zweiten Hohlraum 7 durch ein Vortreiben des Förderkolbens 3 in Richtung des Kartuschenkopfs 2 von dem Förderkolben 3 verschlossen werden und anschließend der Förderkolben 3 und der Austragskolben 4 aufeinander zu getrieben werden, dann bevorzugt aneinander anliegen und das Volumen des zweiten Hohlraums 7 auf null reduziert wird, wenn der Förderkolben 3 weiter in Richtung des Austragskolbens 4 getrieben wird. Diesen Aspekt beherzigend, kann der Fachmann ohne weiteres andere ähnliche oder gleichwirkende Leitungsmittel 8 finden, die als im Rahmen der vorliegenden Erfindung angesehen werden.

Die Austragsöffnung im Kartuschenkopf 2 kann zunächst mit einem Stopfen 12 verschlossen sein (siehe Figuren 1 bis 4 und 7). In dem Stopfen 12 kann ein Durchgang angeordnet sein, durch den Gase aus dem ersten Hohlraum 60 evakuiert und eingeleitet werden können. Um ein Austreten von Zementpulver 5 zu vermeiden, kann vorgesehen sein, dass in dem Durchgang ein Porenfilter 14 angeordnet ist, der für das Zementpulver 5 undurchlässig ist aber für Gase durchlässig ist. An dem Stopfen 12 kann ein farbiges Markierungsmittel 16 in Form eines farbigen Röhrchens vorgesehen sein, an dem eine Bewegung des Stopfens 12 gegen die Austragsöffnung optisch erkennbar ist. Das farbige Markierungsmittel 16 kann hierzu beispielsweise rot sein oder eine andere Signalfarbe haben. Es sind aber auch andere (auch nicht optische) Methoden vorstellbar, eine Bewegung des Stopfens 12 gegen die Austragsöffnung für den Anwender kenntlich zu machen.

An dem Kartuschenkopf 2 kann eine Flügelschraube 20 angeordnet sein, mit der der Stopfen 12 in der Austragsöffnung lösbar befestigt ist. In der Flügelschraube 20 kann eine Bohrung vorgesehen sein, in der der Stopfen 12 gegen die Flügelschraube 20 beweglich sein kann. Sobald das Markierungsmittel 16 (also das Röhrchen) aus der Flügelschraube 20 hervorgedrückt ist, kann der Anwender der Vorrichtung erkennen, dass der Knochenzementteig 62 einsatzbereit ist, da das Zementpulver 5 einen vom Austragskolben 4 vermittelten Druck nicht auf den Porenfilter 14 und den Stopfen 12 vermitteln kann, solange es nicht mit der Monomerflüssigkeit 6 benetzt ist und sich wie ein Fluid verhält.

Um ein Austreten von Monomerflüssigkeit 6 und Knochenzementteig 62 zu verhindern und/oder um den ersten Hohlraum 60 von dem zweiten Hohlraum 7 oder andere Bereiche voneinander abzudichten, können Abdichtungen vorgesehen sein. So können zwei Dichtungsringe 22 in zwei umlaufenden Nuten an einem Außenumfang des Stopfens 12 zum Abdichten gegen die Flügelmutter 20 angeordnet sein. Es kann ein Dichtungsring 24 vorgesehen sein, der in einer umlaufenden Nut an der Außenseite der Kartusche 1 angeordnet ist und den Kartuschenkopf 2 gegen die Kartusche 1 abdichtet. Es können zwei Dichtungsringe 26 in zwei umlaufenden Nuten am Außenumfang des Austragskolbens 4 angeordnet sein, die die Austragskolben 4 gegen die Innenwand der Kartusche 1 abdichten. Es können zwei Dichtungsringe 28 in zwei umlaufenden Nuten am Außenumfang des Förderkolbens 3 angeordnet sein, die den Förderkolben 3 und den Behälter 34 gegen die Innenwand der Kartusche 1 abdichten. Es können zwei Dichtungsringe 29 in zwei umlaufenden Nuten am Außenumfang eines Behälters 34 angeordnet sein, der an der Rückseite des Förderkolbens 3 angeordnet sein kann, wobei die Dichtungsringe 29 den Behälter 34 gegen die Innenwand der Kartusche 1 abdichten.

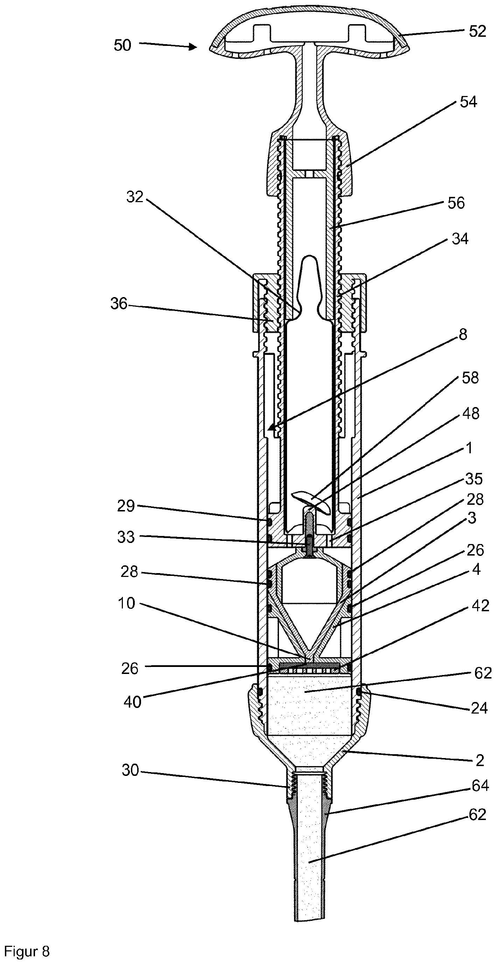

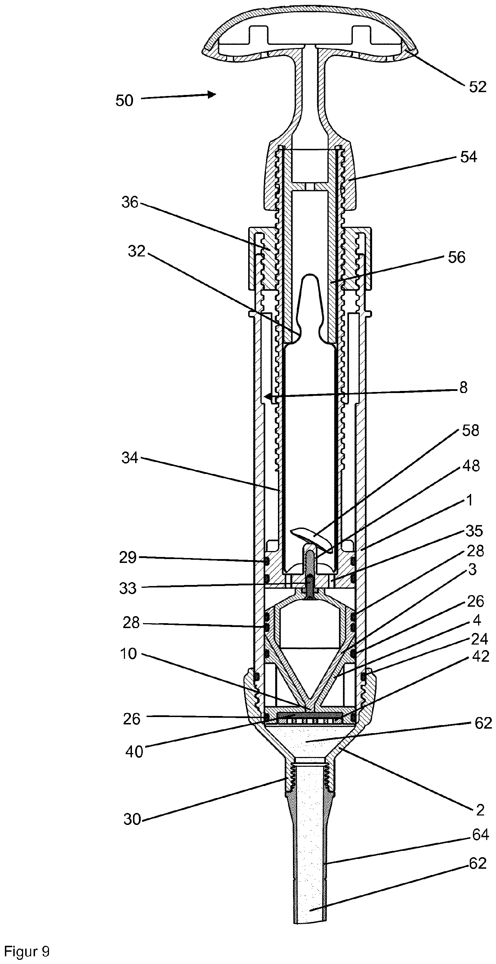

An der Spitze des Kartuschenkopfs 2 kann ein Stutzen 30 ausgebildet sein, der die Austragsöffnung umschließt. In dem Stutzen 30 kann ein Innengewinde vorgesehen sein, in das ein Austragsrohr 64 (siehe Figuren 8 und 9) mit einem passenden Außengewinde eingeschraubt werden kann. In das gleiche Innengewinde oder an einem anderen Befestigungsmittel des Stutzens 30 kann die Flügelmutter 20 mit einem passenden Außengewinde oder einem anderen passenden Gegenbefestigungsmittel an dem Stutzen 30 lösbar befestigt sein.

Um ein vollständiges Full-Prepacked-Mischsystem zu erhalten, kann ein Monomerflüssigkeitsbehälter 32, beispielsweise in Form einer aufbrechbaren Ampulle aus Glas oder Kunststoff, in dem Behälter 34 angeordnet sein, der hierzu an der Rückseite des Förderkolbens 3 angeordnet sein kann oder befestigt sein kann. Beispielsweise kann der Behälter 34 mit einer Schraube, einer Niete oder einem Bolzen an dem Förderkolben 3 befestigt sein, so wie das in den Figuren 1, 2 und 4 bis 9 gezeigt ist. In dem Monomerflüssigkeitsbehälter 32 kann die Monomerflüssigkeit 6 enthalten sein. Der Innenraum des Behälters 34 kann über zumindest eine Monomerleitung 35 mit den rückseitigen Teil 11 des Innenraums der Kartusche 1 flüssigkeitsdurchlässig verbunden sein. An den Einmündungen des Innenraums des Behälters 34 zu der zumindest einen Monomerleitung 35 kann ein Sieb und/oder ein Porenfilter angeordnet sein, mit dem oder denen Bruchstücke des Monomerflüssigkeitsbehälters 32 zurückgehalten werden können. Der rückseitige Teil 11 des Innenraums der Kartusche 1 kann wiederum über das Leitungsmittel 8 (die Nuten in der Kartuscheninnenwand) mit dem zweiten Hohlraum 7 flüssigkeitsdurchlässig verbunden sein. Es kann eine in dem Behälter 34 freigesetzte Monomerflüssigkeit 6 aus dem Behälter 34 in den zweiten Hohlraum 7 fließen, wenn die Vorrichtung mit dem Kartuschenkopf 2 nach unten gehalten oder aufgestellt ist.

An einem dem Kartuschenkopf 2 gegenüberliegenden rückseitigen Ende der Kartusche 1 kann eine Ringhülse 36 mit einem Innengewinde befestigt sein. Diese erleichtert die Montage der Vorrichtung, wenn sie als zur Kartusche 1 separates Teil vorliegt. Die Ringhülse 36 kann hierzu mit einem passenden Außengewinde in ein Innengewinde an dem rückseitigen Ende der Kartusche 1 geschraubt sein.

Es kann gemäß einer bevorzugten Weiterbildung der vorliegenden Erfindung vorgesehen sein, dass der Behälter 34 an seinem Außenumfang ein Außengewinde aufweist. Dieses Außengewinde passt besonders bevorzugt zu dem Innengewinde der Ringhülse 36 oder zu einem Innengewinde an dem rückseitigen Ende der Kartusche 1. Dadurch kann der Behälter 34 in die Kartusche 1 eingeschraubt werden. Dies kann einen Vortrieb des Förderkolbens 3 im Innenraum der Kartusche 1 ermöglichen. Theoretisch kann als Alternative zum Behälter 34 auch ein Zylinder (nicht gezeigt) mit einem passenden Außengewinde an der Rückseite des Förderkolbens 3 befestigt oder auch lose angeordnet sein, so dass durch Einschrauben des Zylinders der Förderkolben 3 und über den Förderkolben 3 auch der Austragskolben 4 im Innenraum der Kartusche 1 in Richtung des Kartuschenkopfs 2 zu drücken ist. Ein dem Kartuschenkopf 2 zugewandter vorderer Teil des Behälters 34 hat bevorzugt kein Außengewinde und kann so in der Ringhülse 36 und damit in den Innenraum der Kartusche 1 hineingeschoben werden, bis das Außengewinde des Behälters 34 auf das Innengewinde an der Ringhülse 36 trifft. Die Länge des Bereichs des Behälters 34 ohne Außengewinde und die Länge des Leitungsmittels 8 können bevorzugt so aufeinander abgestimmt sein, dass der Förderkolben 3 das zumindest eine Leitungsmittel 8 gegen den zweiten Hohlraum 7 abdichtet, bevor das Außengewinde des Behälters 34 in das Innengewinde des Ringhülse 36 beziehungsweise der Kartusche 1 greift.

Um eine vorzeitige oder ungewollte Bewegung des Behälters 34 und/oder der Ringhülse 36 gegen die Kartusche 1 zu verhindern, kann ein lösbares Sicherungselement (nicht gezeigt) vorgesehen sein. An einer dem Kartuschenkopf 2 abgewandten Rückseite des Behälters 34 kann ein Öffnungsmittel 50 zum Öffnen des Monomerflüssigkeitsbehälters 32 angeordnet sein. Um ein versehentliches Öffnen des Monomerflüssigkeitsbehälters 32 zu verhindern, kann ein abziehbares Sicherungsmittel 38 vorgesehen sein. Das Sicherungsmittel 38 kann hierzu als Spange geformt sein, die am Außenumfang des Behälters 34 oder einer anderen rückseitigen Verlängerung des Förderkolbens 3 eingreift und sich an einer Vorderseite des Öffnungsmittels 50 abstützt.

Es kann bevorzugt vorgesehen sein, dass in oder an dem Austragskolben 4 ein Porenfilter 40 angeordnet ist. Mit dem Porenfilter 40 kann die Durchführung 9 abgedeckt sein. Hierdurch wird verhindert, dass Zementpulver 5 aus dem ersten Hohlraum 60 in die Durchführung 9 oder in den zweiten Hohlraum 7 eindringt, dort bei Reaktion mit der Monomerflüssigkeit 6 eine gelartige Barriere bildet und so einer Verteilung der Monomerflüssigkeit 6 in dem Zementpulver 5 entgegenwirkt.

Der Porenfilter 40 kann bevorzugt eine Kreisscheibe sein. Der Porenfilter 40 kann mit einer Scheibe 42 abgedeckt und in dem Austragskolben 4 befestigt sein. Um eine Durchleitung und Verteilung der Monomerflüssigkeit 6 in den ersten Hohlraum 60 und in dem Zementpulver 5 zu ermöglichen beziehungsweise zu erleichtern, können in der Scheibe 42 eine Vielzahl von Bohrungen oder Löchern vorgesehen sein (siehe Figur 2).

Im Innenraum des Behälters 34 kann ein Stift 48 oder ein Stechdorn oder eine Scheide angeordnet sein. Der Stifte 48, der Stechdorn oder die Schneide kann an einem Kartuschenboden 58 des Monomerflüssigkeitsbehälters 32 anliegen und dazu verwendet werden, den Monomerflüssigkeitsbehälter 32 am Kartuschenboden 58 aufzubrechen oder eine Monomerflüssigkeitsbehälter 32 aufzustechen oder aufzuschneiden. Bevorzugt kann die Stift 48 im Bereich der Einmündung in die Monomerleitung 35 angeordnet sein.

An der vom Förderkolben 3 abgewandten Rückseite des Behälters 34 kann das Öffnungsmittel 50 zum Öffnen des Monomerflüssigkeitsbehälters 32 angeordnet sein. Das Öffnungsmittel 50 kann hierzu einen manuell bedienbaren Griff 52 und eine Kappe 54 aufweisen. Es kann vorgesehen sein, dass die Kappe 54 gegen den Behälter 34 beweglich gelagert ist, wobei vorzugsweise die Kappe 54 auf das Außengewinde des Behälters 34 aufschraubbar ist. Der Behälter 34 kann an seiner Rückseite offen sein. Im Inneren des Behälters 34 kann eine Hülse 56 in Form eines Rohrstücks als Teil des Öffnungsmittels 50 beweglich gegen den Behälter 34 gelagert sein. Durch eine Bewegung der Hülse 56 in den Behälter 34 hinein kann der Monomerflüssigkeitsbehälter 32 in dem Behälter 34 geöffnet werden. Bevorzugt wird eine Ampulle als Monomerflüssigkeitsbehälter 32 mit dem Kartuschenboden 58 derart auf den Stift 48 gedrückt, dass der Kartuschenboden 58 aufbricht oder abbricht und dadurch die Ampulle geöffnet ist (siehe Figur 4). Auf diese Weise kann die Monomerflüssigkeit 6 aus dem Monomerflüssigkeitsbehälter 32 im Inneren des Behälters 34 freigesetzt werden.

Im Folgenden wird der Ablauf eines erfindungsgemäßen Verfahrens am Beispiel der erfindungsgemäßen Vorrichtung anhand der Figuren 1 bis 9 erläutert.

Die Vorrichtung befindet sich zunächst im Ursprungszustand oder Lagerungszustand, wie er in den Figuren 1 bis 3 gezeigt ist. In diesem Zustand kann die Vorrichtung durch geeignete Öffnungen evakuiert und mit Ethylenoxid sterilisiert werden.

Als nächstes kann die Vorrichtung mit dem Kartuschenkopf 2 nach unten gehalten werden. Dann kann das Sicherungsmittel 38 abgezogen werden. Danach kann das Öffnungsmittel 50 auf den Behälter 34 geschraubt werden, bis der Kartuschenboden 58 des Monomerflüssigkeitsbehälters 32 aufbricht. Durch das Aufbrechen des Monomerflüssigkeitsbehälters 32 wird die darin enthaltene Monomerflüssigkeit 6 freigesetzt und kann aus dem Behälter 34 durch die zumindest eine Monomerleitung 35, durch den rückseitigen Teil 11 des Innenraums der Kartusche 1 und durch das Leitungsmittel 8 in den zweiten Hohlraum 7 fließen. Diese Situation ist in Figur 4 dargestellt.

Anschließend kann, sofern vorhanden, das Sicherungselement abgezogen werden. Der Behälter 34 kann nun tiefer in die Kartusche 1 eingeschoben werden, bis das Außengewinde am Behälter 34 auf das Innengewinde an der Ringhülse 36 trifft und eine weitere Bewegung des Behälters 34 in die Kartusche 1 hinein blockiert. Diese Situation ist in Figur 5 dargestellt. Die Monomerflüssigkeit 6 ist dann bereits vollständig oder sehr weitgehend in den zweiten Hohlraum 7 geflossen. Der Förderkolben 3 ist in dieser Situation bereits derart weit über das Leitungsmittel 8 geschoben, dass der Förderkolben 3 die Verbindung des Leitungsmittels 8 in den zweiten Hohlraum 7 abdichtet.

In dieser Situation kann die Vorrichtung umgedreht werden, so dass der Kartuschenkopf 12 nach oben weist oder in etwa nach oben weist (beispielweise mit einem Winkel von weniger als 50° zum Lot nach oben weist). Die Vorrichtung kann dann mit dem Kartuschenkopf 2 nach oben gehalten werden, damit Gaseinschlüsse beziehungsweise Lufteinschlüsse aus dem zweiten Hohlraum 7 durch die Durchführung 9 und durch das Zementpulver 5 im ersten Hohlraum 60 nach oben durch den Stopfen 12 aus der Vorrichtung austreten und ausgepresst werden kann. Durch die trichterförmige Oberfläche 44 an der Rückseite des Austragskolbens 4 sammeln sich die Gas- beziehungsweise Lufteinschlüsse in deren Spitze und können so leicht und auf einfache Weise durch die Durchführung 9 im Austragskolben 4 in den ersten Hohlraum 60 gepresst werden, indem der Förderkolben 3 in Richtung des Austragskolbens 4 gedrückt wird. Mit dem Behälter 34 kann auch der Förderkolben 3 in dem Innenraum der Kartusche 1 in Richtung des Austragskolbens 4 gedrückt werden. Das Volumen des zweiten Hohlraums 7 kann durch die Bewegung des Förderkolbens 3 in Richtung des Austragskolbens 4 verringert werden.

Die Ringhülse 36 kann nun nach oben von dem Kartuschenkopf 10 der Kartusche 1 weg geschraubt werden, bis das Innengewinde der Ringhülse 36 in das Außengewinde des Behälters 34 greift. Währenddessen kann die Monomerflüssigkeit 6 bereits beginnen, durch die Durchführung 9 im Austragskolben 4 und gegebenenfalls durch die Porenscheibe 40 und die Scheibe 42 hindurch in den ersten Hohlraum 60 fließen. Diese Situation ist in Figur 6 dargestellt, bei der die Vorrichtung im Vergleich zu Figur 5 um 180° gedreht ist, so dass der Kartuschenkopf 2 (in den Figuren 5 und 6 nicht zu sehen) nach oben ausgerichtet ist, um das Entweichen von Gas oder Luft aus dem ersten Hohlraum 60 und dem zweiten Hohlraum 7 zu ermöglichen beziehungsweise zu erleichtern. Um die Vorrichtung nicht exakt lotrecht mit dem Kartuschenkopf 2 nach oben halten zu müssen, hat die trichterförmige Oberfläche 44 eine derart steile Neigung (über 45°), dass auch bei einer Stellung der Kartusche 1 mit einer Neigung von 45° noch eine Steigung zur Durchführung 9 hin vorhanden ist, so dass Gaseinschlüsse beziehungsweise Luftblasen in der trichterförmigen Oberfläche 44 nach oben zur Durchführung 9 steigen und dort aus dem zweiten Hohlraum 7 durch die Durchführung 9 in den ersten Hohlraum 60 entweichen. Von dort aus treibt die nachfließende Monomerflüssigkeit 6 das Gas beziehungsweise die Luft durch die Pulverteilchen des Zementpulvers 5 und durch den Porenfilter 14 aus der Vorrichtung heraus.

Sobald die Monomerflüssigkeit 6 in den ersten Hohlraum 60 fließt, verteilt sie sich aufgrund von Kapillarkräften zwischen den Pulverteilchen des Zementpulvers 5 und dadurch in dem Zementpulver 5. Dabei kann Gas beziehungsweise Luft in den Zwischenräumen zwischen den Pulverpartikeln des Zementpulvers 5 nach oben aus dem Zementpulver 5 verdrängt werden und aus der Austragsöffnung durch den für Gase durchlässigen Porenfilter 14 entweichen. Als nächstes kann der Behälter 34 tiefer in die Kartusche 1 eingeschraubt werden, in dem der Behälter 34 gegen die Ringhülse 36 beziehungsweise gegen die Kartusche 1 gedreht wird.

Der Behälter 34 kann nun noch weiter in die Kartusche 1 eingeschraubt werden. Dabei wird die Monomerflüssigkeit 6 aus dem zweiten Hohlraum 7 in das Zementpulver 5 im ersten Hohlraum 60 gepresst und verteilt sich dort. Schließlich kann der Förderkolben 3 auf den Austragskolben 4 auftreffen, so dass der Förderkolben 3 mit seiner kegelförmigen Oberfläche 46 an der passenden trichterförmigen Oberfläche 44 an der Rückseite des Austragskolbens 4 anliegt, insbesondere flächenbündig anliegt. Währenddessen quilt das Zementpulver 5 mit der Monomerflüssigkeit 6 an und bildet den blasenfreien oder blasenarmen Knochenzementteig 62.

Der Behälter 32 kann noch tiefer in die Kartusche 1 eingeschraubt werden, wenn der Knochenzement 62 bis zur Austragsöffnung gebildet ist beziehungsweise das Zementpulver 5 bis zur Austragsöffnung mit der Monomerflüssigkeit 6 benetzt ist, da erst dann ein fließfähiges Material im ersten Hohlraum 60 erhalten wird, das in die Austragsöffnung gedrückt werden kann. Durch die Bewegung des Knochenzementteigs 62 kann der Stopfen 12 in der Austragsöffnung bewegt werden. Dies kann durch eine Verschiebung des Markierungsmittels 16 aus dem Stopfen 12 heraus optisch erkannt werden, so dass der Anwender weiß, dass der Knochenzementteig 62 nun einsatzbereit ist. Diese Situation ist in Figur 7 dargestellt.

Als nächstes kann die Flügelmutter 20 mit dem Stopfen 12 aus der Austragsöffnung geschraubt werden. Dann kann das Austragsrohr 64 in den Stutzen 30 geschraubt werden. Sobald der vortretende Stopfen 12 anzeigt, dass der Knochenzementteig 62 einsatzbereit ist, kann die Vorrichtung wieder umgedreht werden beziehungsweise in einer beliebigen Stellung gehalten werden, da die Luft- beziehungsweise Gaseinschlüsse nun aus dem ersten Hohlraum 60 entwichen sind und so ein blasenfreier Knochenzementteig 62 entstehen kann. Daran anschließend kann der Knochenzementteig 62 durch weiteres Einschrauben des Behälters 34 und damit einhergehend durch weiteres Vortreiben des Förderkolbens 3 und des Austragskolbens 4 in Richtung des Austragsöffnung aus der Austragsöffnung ausgetragen werden. Diese Situation ist in Figur 8 dargestellt, bei der die Vorrichtung wieder mit dem Kartuschenkopf 2 nach unten gehalten gezeigt ist. Der Knochenzementteig 62 kann durch weiteres Einschrauben des Behälters 34 in die Kartusche 1 ausgetrieben, indem der Förderkolben 3 und der Austragskolben 4 durch die Bewegung des Behälters angetrieben werden, bis der Austragskolben 4 auf den Kartuschenkopf 2 trifft. Diese Situation ist in Figur 9 dargestellt. Durch die kegelförmige Vertiefung im Kartuschenkopf 2 kann im Kartuschenkopf 2 ein Totvolumen entstehen, in dem ein Teil des Knochenzementteigs 62 zurückgehalten wird. Aufgrund von elastischen Eigenschaften der Vorrichtung kann es sein, dass am Ende des Auspressvorgangs noch geringe Mengen der Monomerflüssigkeit 6 in diesen Bereich gepresst werden. Durch das Totvolumen kann verhindert werden, dass dieser Teil des Knochenzementteigs 62, der dann eine etwas andere Konsistenz hat als der restliche Knochenzementteig 62, in der Vorrichtung zurückgehalten werden kann.

### Bezugszeichenliste

- 1: Kartusche

- 2: Kartuschenkopf
- 3: Förderkolben
- 4: Austragskolben
- 5: Zementpulver
- 6: Monomerflüssigkeit
- 7: Zweiter Hohlraum
- 8: Leitungsmittel
- 9: Durchführung
- 10: Stift
- 11: Rückseitiger Teil des Innenraums der Kartusche
- 12: Stopfen
- 14: Porenfilter
- 16: Markierungsmittel
- 20: Flügelschraube
- 22: Dichtungsring
- 24: Dichtungsring
- 26: Dichtungsring
- 28: Dichtungsring
- 29: Dichtungsring
- 30: Stutzen
- 32: Monomerflüssigkeitsbehälter
- 33: Schraube
- 34: Behälter
- 35: Monomerleitung
- 36: Ringhülse
- 38: Sicherungsmittel
- 40: Porenfilter
- 42: Scheibe
- 44: Trichterförmige Oberfläche
- 46: Kegelförmige Oberfläche
- 48: Stift
- 50: Öffnungsmittel
- 52: Griff
- 54: Kappe
- 56: Hülse
- 58: Kartuschenboden
- 60: Erster Hohlraum
- 61: Verdickung
- 62: Knochenzementteig
- 64: Austragsrohr

## Patentansprüche

1. Vorrichtung zum Herstellen eines Knochenzementteigs (62) aus einer Monomerflüssigkeit (6) und einem Zementpulver (5) als Ausgangskomponenten des Knochenzementteigs (62) und zum Austragen des Knochenzementteigs (62), die Vorrichtung aufweisend
eine Kartusche (1) mit einem zylindrischen Innenraum,
einen Kartuschenkopf (2) mit einer Austragsöffnung zum Austreiben des Knochenzementteigs (62), wobei der Kartuschenkopf (2) die Kartusche (1) an einer Vorderseite der Kartusche (1) bis auf die Austragsöffnung verschließt,
einen Förderkolben (3), der im Innenraum der Kartusche (1) angeordnet ist und der im Innenraum der Kartusche (1) in Richtung der Austragsöffnung drückbar gelagert ist, einen Austragskolben (4), der im Innenraum der Kartusche (1) zwischen der Austragsöffnung und dem Förderkolben (3) angeordnet ist und der im Innenraum der Kartusche (1) in Richtung der Austragsöffnung drückbar gelagert ist, wobei eine dem Förderkolben (3) zugewandte Rückseite des Austragskolbens (4) eine sich in Richtung des Kartuschenkopfs (2) verjüngende Vertiefung aufweist,
einen ersten Hohlraum (60), der von dem Kartuschenkopf (2), von Innwänden der Kartusche (1) und von dem Austragskolben (4) begrenzt ist, wobei das Zementpulver (5) in dem ersten Hohlraum (60) angeordnet ist,
einen zweiten Hohlraum (7), der ein Teil des zylindrischen Innenraums der Kartusche (1) ist, wobei der zweite Hohlraum (7) von dem Austragskolben (4) und dem Förderkolben (3) begrenzt ist,
einen rückseitigen Teil (11) des Innenraums der Kartusche (1), dessen Vorderseite von einer von dem Austragskolben (4) abgewandten Rückseite des Förderkolbens (3) begrenzt ist,
ein Leitungsmittel (8), das den zweiten Hohlraum (7) mit dem rückseitigen Teil (11) des Innenraums der Kartusche (1) an dem Förderkolben (3) vorbei und für die Monomerflüssigkeit (6) durchlässig verbindet, wobei das Leitungsmittel (8) durch ein Verschieben des Förderkolbens (3) in Richtung des Austragskolbens (4) von dem Förderkolben (3) gegen den zweiten Hohlraum (7) verschließbar ist, und
eine Durchführung (9), wobei die Durchführung (9) an einer Spitze der sich verjüngenden Vertiefung beginnt, sich durch den Austragskolben (4) erstreckt und den ersten Hohlraum (60) und den zweiten Hohlraum (7) für die Monomerflüssigkeit (6) durchlässig aber für das Zementpulver (5) undurchlässig miteinander verbindet.

2. Vorrichtung nach Anspruch 1, **dadurch gekennzeichnet, dass**
eine dem Austragskolben (4) zugewandte Vorderseite des Förderkolbens (3) eine sich in Richtung des Austragskolbens (4) vorspringende Oberfläche aufweist, die sich vorzugsweise in Richtung des Austragskolbens (4) stetig verjüngt.

3. Vorrichtung nach Anspruch 1 oder 2, **dadurch gekennzeichnet, dass**
die sich in Richtung des Kartuschenkopfs (2) verjüngende Vertiefung eine sich von der Innenwand der Kartusche (1) aus stetig verjüngende Vertiefung in Form einer trichterförmigen Oberfläche (44) ist, wobei bevorzugt eine vorspringende Oberfläche an der Vorderseite des Förderkolbens (3) in die trichterförmige Oberfläche (44) an der Rückseite des Austragskolbens (4) einschiebbar ist, wobei besonders bevorzugt die Durchführung (9) im Austragskolben (4) an der tiefsten Stelle der trichterförmigen Oberfläche (44) in den Austragskolben (4) mündet.

4. Vorrichtung nach einem der vorangehenden Ansprüche, **dadurch gekennzeichnet, dass**
eine dem Austragskolben (4) zugewandte Vorderseite des Förderkolbens (3) und die dem Förderkolben (3) zugewandte Rückseite des Austragskolbens (4) zueinander flächenbündig passende Formen aufweisen, so dass die Vorderseite des Förderkolbens (3) an der Rückseite des Austragskolbens (4) flächenbündig anliegt, wenn der Förderkolben (3) gegen den Austragskolben (4) gedrückt ist, wobei vorzugsweise das Volumen des zweiten Hohlraums (7) dadurch auf maximal 1% des Volumens des zweiten Hohlraums (7) in einem Ausgangszustand reduzierbar ist.

5. Vorrichtung nach einem der vorangehenden Ansprüche, **dadurch gekennzeichnet, dass**
die dem Förderkolben (3) zugewandte Rückseite des Austragskolbens (4) eine trichterförmige Oberfläche (44) als die sich verjüngende Vertiefung aufweist, in deren tiefsten Punkt die Durchführung (9) angeordnet ist, und eine dem Austragskolben (4) zugewandte Vorderseite des Förderkolbens (3) eine vorspringende kegelförmige Oberfläche (46) mit der gleichen Steigung wie die trichterförmige Oberfläche (44) an der Rückseite des Austragskolbens (4) aufweist, wobei vorzugsweise an der Spitze der vorspringenden kegelförmigen Oberfläche (46) an der Vorderseite des Austragskolbens (4) ein zylindrischer Stift (10) angeordnet ist, der in eine zylindrische Vertiefung als Teil der Durchführung (9) im Austragskolben (4) einsteckbar ist.

6. Vorrichtung nach Anspruch 5, **dadurch gekennzeichnet, dass** die trichterförmige Oberfläche (44) an der Rückseite des Austragskolbens (4) einen Steigungswinkel von mindestens 45° aufweist, bevorzugt von mindestens 55°, besonders bevorzugt von mindestens 60°, und
die kegelförmige Oberfläche (46) an der Vorderseite des Förderkolbens (3) einen dazu passenden Steigungswinkel von mindestens 45° aufweist, bevorzugt von mindestens 55°, besonders bevorzugt von mindestens 60°aufweist.

7. Vorrichtung nach einem der vorangehenden Ansprüche, **dadurch gekennzeichnet, dass**
an der Rückseite des Förderkolbens (3) ein Behälter (34) angeordnet ist, in dem ein Monomerflüssigkeitsbehälter (32) enthaltend die Monomerflüssigkeit (6) angeordnet ist, insbesondere eine Ampulle aus Glas oder Kunststoff, wobei der Monomerflüssigkeitsbehälter (32) innerhalb des Behälters (34) zu öffnen ist und wobei der Behälter (34) über zumindest eine Monomerleitung (35) mit dem rückseitigen Teil (11) des Innenraums der Kartusche (1) flüssigkeitsdurchlässig verbunden ist, wobei bevorzugt ein Öffnungsmittel (50) an einer dem Förderkolben (3) gegenüberliegende Seite des Behälters (34) angeordnet ist, mit dem der Monomerflüssigkeitsbehälter (32) innerhalb des Behälters (34) zu öffnen ist, wobei besonders bevorzugt das Öffnungsmittel (50) eine an einer Kappe (54) befestigte Hülse (56) ist, wobei die Kappe (54) auf ein Gewinde des Behälters (34) schraubbar ist und die Kappe (54) hierzu ein Gegengewinde aufweist, so dass beim Aufschrauben der Kappe (54) der Monomerflüssigkeitsbehälter (32), insbesondere die Ampulle, mit der Hülse (56) auf zumindest einen vorstehenden Stift (48) an der Innenseite des Behälters (34) gedrückt wird und dadurch der Monomerflüssigkeitsbehälter (32), insbesondere die Ampulle, aufbrechbar ist.

8. Vorrichtung nach Anspruch 7, **dadurch gekennzeichnet, dass**
der Behälter (34) ein Außengewinde aufweist, das in ein Innengewinde an einem dem Kartuschenkopf (2) gegenüberliegenden Ende der Kartusche (1) schraubbar ist, wobei durch Einschrauben des Behälters (34) in die Kartusche (1) der Förderkolben (3) in Richtung der Austragsöffnung drückbar ist und der Austragskolben (4) mit dem Förderkolben (3) in Richtung der Austragsöffnung drückbar ist, wobei bevorzugt das Innengewinde Teil einer Ringhülse (36) ist, die an dem dem Kartuschenkopf (2) gegenüberliegenden Ende der Kartusche (1) mit der Kartusche (1) verbunden ist, und/oder
die Außenseite des Behälters (34) in einem ersten Abschnitt ausgehend von einer dem Kartuschenkopf (2) zugewandten Stirnseite kein Außengewinde besitzt und in einem zweiten Abschnitt ein Außengewinde besitzt.

9. Vorrichtung nach einem der vorangehenden Ansprüche, **dadurch gekennzeichnet, dass**
das Leitungsmittel (8) an einer Stelle an der Innenwand der Kartusche (1) endet oder einmündet, die von dem Austragskolben (4) derart axial bezogen auf die Achse des zylindrischen Innenraums derart beabstandet ist, dass das Volumen des zweiten Hohlraums (7) mindestens so groß ist wie das Volumen der durch das Leitungsmittel (8) eingeleiteten Monomerflüssigkeit (6), insbesondere der in dem Monomerflüssigkeitsbehälter enthaltenen Monomerflüssigkeit (6), wenn der Förderkolben (3) gerade so weit in Richtung des Austragskolbens (4) verschoben ist, dass der Förderkolben (3) das Leitungsmittel (8) gegen der zweiten Hohlraum (7) verschließt.

10. Vorrichtung nach einem der vorangehenden Ansprüche, **dadurch gekennzeichnet, dass**
ein Stopfen (12) in der Austragsöffnung angeordnet ist, der die Austragsöffnung für das Zementpulver (5) undurchlässig verschließt, insbesondere für Gase durchlässig verschließt, wobei vorzugsweise der Stopfen (12) in der Austragsöffnung beweglich angeordnet ist, so dass der Stopfen (12) durch einen Druck auf den fertig gemischten Knochenzementteig (62) aus der Austragsöffnung heraus gedrückt werden kann, wobei besonders bevorzugt ein von außen sichtbares Markierungsmittel (16) an dem Stopfen (12) befestigt ist.

11. Vorrichtung nach einem der vorangehenden Ansprüche, **dadurch gekennzeichnet, dass**
die Durchführung (9) durch ein für Gase und Flüssigkeiten undurchlässigen Stopfen verschlossen ist, wobei der Stopfen in der Durchführung (9) in Richtung Kartuschenkopf (2) verschiebbar ist, wobei bevorzugt an der Vorderseite des Förderkolbens (3) ein Stift (10) angeordnet ist, mit dem der Stopfen in der Durchführung (9) aus der Durchführung (9) herausdrückbar ist und damit die Durchführung (9) zu öffnen ist, wobei besonders bevorzugt, der Stift (10) eine solche axiale Erstreckung bezüglich des zylindrischen Innenraums der Kartusche (1) hat, dass bei einer Berührung des Stifts (10) mit dem Stopfen, das Volumen zwischen der Rückseite des Austragskolbens (4) und der Vorderseite des Förderkolbens (3) im Innenraum der Kartusche (1) zumindest genauso groß ist, wie das Volumen der Monomerflüssigkeit (6) in einem Monomerflüssigkeitsbehälter (32), der in der Vorrichtung angeordnet ist oder anzuordnen ist.

12. Verfahren zur Herstellung eines Knochenzementteigs (62), insbesondere eines pastenförmigen Polymethylmethacrylat-Knochenzementteigs, wobei der Knochenzementteig (62) aus einem Zementpulver (5) und einer Monomerflüssigkeit (6) mit einer Vorrichtung nach einem der vorangehenden Ansprüche hergestellt wird, **gekennzeichnet durch** die folgenden nacheinander ablaufenden Schritte:
A) Einleiten der Monomerflüssigkeit (6) durch das Leitungsmittel (8) in den zweiten Hohlraum (7),
B) Drücken des Förderkolbens (3) in Richtung des Austragskolbens (4), bis der Förderkolben (3) alle Verbindungen des Leitungsmittels (8) zum zweiten Hohlraum (7) verschließt,
C) Halten der Vorrichtung mit dem Kartuschenkopf (2) nach oben und weiteres Drücken des Förderkolbens (3) in Richtung des Austragskolbens (4), wobei Luft oder Gas aus dem ersten Hohlraum (60) und aus dem zweiten Hohlraum (7) durch den Kartuschenkopf (2) entfernt wird und wobei die Monomerflüssigkeit (6) aus dem zweiten Hohlraum (7) in das Zementpulver (5) in den ersten Hohlraum (60) gedrückt wird,
D) Drücken des Austragskolbens (4) mit dem Förderkolben (3) in Richtung der Austragsöffnung, wobei der in dem ersten Hohlraum (60) gebildete Knochenzementteig (62) durch die Austragsöffnung ausfließt.

13. Verfahren nach Anspruch 12, **dadurch gekennzeichnet, dass**
die Vorrichtung in Schritt A), und vorzugsweise auch in Schritt B), mit dem Kartuschenkopf (2) nach unten aufgestellt oder gehalten wird und in Schritt C) Gaseinschlüsse aus dem zweiten Hohlraum (7) durch das die Durchführung (9), den ersten Hohlraum (60) und die Austragsöffnung im Kartuschenkopf (2) entweichen, vorzugsweise durch einen für Gase durchlässigen aber für das Zementpulver (5) undurchlässigen Filter in der Austragsöffnung entweichen, wobei der Filter die Austragsöffnung für Gase durchlässig und für das Zementpulver (5) undurchlässig verschließt.

14. Verfahren nach Anspruch 12 oder 13, **dadurch gekennzeichnet, dass** in Schritt D) der Förderkolben (3) flächenbündig an dem Austragskolben (4) anliegt und bevorzugt in Schritt C) das Volumen des zweiten Hohlraums (7) vollständig oder bis auf maximal 1% reduziert wird, und/oder
in Schritt D) durch den auf den Knochenzementteig (62) wirkenden Druck ein Stopfen (12) in der Austragsöffnung bewegt oder hervorgedrückt wird, wobei bevorzugt hierauf der Stopfen (12) aus der Austragsöffnung entfernt wird und besonders bevorzugt danach ein Applikationsrohr (64) an dem Kartuschenkopf (2) der Kartusche (1) befestigt wird.

15. Verfahren nach einem der Ansprüche 12 bis 14, **dadurch gekennzeichnet, dass** vor Schritt A) ein Monomerflüssigkeitsbehälter (32) enthaltend die Monomerflüssigkeit (6) in einem Behälter (34) geöffnet wird und die Monomerflüssigkeit (6) in dem Behälter (34) freigesetzt wird, wobei der Behälter (34) an einer dem Austragskolben (4) abgewandten Rückseite des Förderkolbens (3) angeordnet ist, insbesondere befestigt ist, und die Monomerflüssigkeit (6) in Schritt A) aus dem Behälter (34) durch das Leitungsmittel (8) in den zweiten Hohlraum (7) fließt, wobei vorzugsweise in Schritt B) und C) der Förderkolben (3) und in Schritt D) der Förderkolben (3) und der Austragskolben (4) durch Eindrücken oder Einschrauben des Behälters (34) in die Kartusche (1) angetrieben werden.

## Claims

1. Device for the production of a bone cement dough (62) from a monomer liquid (6) and a cement powder (5) as starting components of the bone cement dough (62), and for dispensing of the bone cement dough (62), the device comprising
a cartridge (1) with a cylindrical internal space;
a cartridge head (2) with a dispensing opening for dispensing the bone cement dough (62), whereby the cartridge head (2) closes the cartridge (1) on a front side of the cartridge (1) except for the dispensing opening;
a conveying plunger (3) that is arranged in the internal space of the cartridge (1) and is supported in the internal space of the cartridge (1) such that it is pushable in the direction of the dispensing opening;
a dispensing plunger (4) that is arranged in the internal space of the cartridge (1) between the dispensing opening and the conveying plunger (3) and that is supported in the internal space of the cartridge (1) such that it is pushable in the direction of the dispensing opening, whereby a rear side of the dispensing plunger (4) facing the conveying plunger (3) comprises a depression that tapers in the direction of the cartridge head (2);
a first hollow space (60) that is bordered by the cartridge head (2), by internal walls of the cartridge (1), and by the dispensing plunger (4), whereby the cement powder (5) is arranged in the first hollow space (60);
a second hollow space (7) that is part of the cylindrical internal space of the cartridge (1), whereby the second hollow space (7) is bordered by the dispensing plunger (4) and the conveying plunger (3);
a rear-side part (11) of the internal space of the cartridge (1), whose front side is bordered by a rear side of the conveying plunger (3) that faces away from the dispensing plunger (4);
a conducting means (8), which connects the second hollow space (7) to the rear-side part (11) of the internal space of the cartridge (1), by-passing the conveying plunger (3), such as to be permeable to the monomer liquid (6), whereby the conducting means (8) is closable by the conveying plunger (3) with respect to the second hollow space (7) through a shift of the conveying plunger (3) in the direction of the dispensing plunger (4); and
a feedthrough (9), whereby the feedthrough (9) commences at a tip of the tapering depression, extends through the dispensing plunger (4) and connects the first hollow space (60) and the second hollow space (7) such as to be permeable to the monomer liquid (6), but impermeable to the cement powder (5).

2. Device according to claim 1, **characterised in that**
a front side of the conveying plunger (3) that faces the dispensing plunger (4) comprises a surface that projects in the direction of the dispensing plunger (4) and is preferred to taper steadily in the direction of the dispensing plunger (4).

3. Device according to claim 1 or 2, **characterised in that**
the depression that tapers in the direction of the cartridge head (2) a depression in the form of a funnel-shaped surface (44) that tapers steadily originating from the internal wall of the cartridge (1), whereby a projecting surface on the front side of the conveying plunger (3) can preferably be inserted into the funnel-shaped surface (44) on the rear side of the dispensing plunger (4), whereby the feedthrough (9) in the dispensing plunger (4) particularly preferably merges into the dispensing plunger (4) at the lowest site of the funnel-shaped surface (44).

4. Device according to any one of the preceding claims, **characterised in that**
a front side of the conveying plunger (3) that faces the dispensing plunger (4) and the rear side of the dispensing plunger (4) that faces the conveying plunger (3) are shaped with matching surfaces with respect to each other such that the front side of the conveying plunger (3) touches against the rear side of the dispensing plunger (4) with matching surfaces, when the conveying plunger (3) is pushed against the dispensing plunger (4), whereby preferably the volume of the second hollow space (7) thereby is reducible to a maximum of 1% of the volume of the second hollow space (7) in a starting state.

5. Device according to any one of the preceding claims, **characterised in that**
the rear side of the dispensing plunger (4) that faces the conveying plunger (3) comprises a funnel-shaped surface (44) as the tapering depression that has the feedthrough (9) arranged in its lowest site, and a front side of the conveying plunger (3) that faces the dispensing plunger (4) comprises a projecting cone-shaped surface (46) with the same slope as the funnel-shaped surface (44) on the rear side of the dispensing plunger (4), whereby a cylindrical pin (10) that is insertable into a cylindrical depression as part of the feedthrough (9) in the dispensing plunger (4) is preferred to be arranged on the tip of the projecting cone-shaped surface (46) on the front side of the dispensing plunger (4).

6. Device according to claim 5, **characterised in that**
the funnel-shaped surface (44) on the rear side of the dispensing plunger (4) comprises an angle of slope of at least 45°, preferably of at least 55°, particularly preferably of at least 60°, and
the cone-shaped surface (46) on the front side of the conveying plunger (3) comprises a matching angle of slope of at least 45°, preferably of at least 55°, particularly preferably of at least 60°.

7. Device according to any one of the preceding claims, **characterised in that**
a container (34) is arranged on the rear side of the conveying plunger (3), with a monomer liquid container (32) containing the monomer liquid (6) being arranged in the container (34), in particular an ampoule made of glass or plastics, whereby the monomer liquid container (32) is openable inside the container (34) and whereby the container (34) is connected to the rear-side part (11) of the internal space of the cartridge (1) in liquid-permeable manner by means of at least one monomer line (35), whereby an opening means (50) is preferably arranged on a side of the container (34) opposite from the conveying plunger (3), by means of which the monomer liquid container (32) is openable inside the container (34), whereby the opening means (50) is particularly preferred to be a sleeve (56) attached to a cap (54), whereby the cap (54) is screwable onto a thread of the container (34) and the cap (54) comprises a counter-thread for this purpose such that, when the cap (54) is being screwed on, the monomer liquid container (32), in particular the ampoule, is pushed, by the sleeve (56), onto at least one projecting pin (48) on the inside of the container (34) and thus the monomer liquid container (32), in particular the ampoule, is breakable open.

8. Device according to claim 7, **characterised in that**
the container (34) comprises an external thread that is screwable into an internal thread on an end of the cartridge (1) opposite from the cartridge head (2), whereby the conveying plunger (3) is pushable in the direction of the dispensing opening by screwing the container (34) into the cartridge (1), and the dispensing plunger (4) is pushable by the conveying plunger (3) in the direction of the dispensing opening, whereby the internal thread preferably is part of a ring sleeve (36) that is connected to the cartridge (1) on the end of the cartridge (1) opposite from the cartridge head (2), and/or
the external side of the container (34) possesses no external thread in a first section that originates from a front side that faces the cartridge head (2), and possesses an external thread in a second section.

9. Device according to any one of the preceding claims, **characterised in that**
the conducting means (8) terminates or merges at a site on the internal wall of the cartridge (1) that is situated at an appropriate axial distance from the dispensing plunger (4) relative to the axis of the cylindrical internal space, such that the volume of the second hollow space (7) is at least equal to the volume of the monomer liquid (6) conducted into it through the conducting means (8), in particular of the monomer liquid (6) contained in the monomer liquid container, when the conveying plunger (3) is pushed just far enough in the direction of the dispensing plunger (4) such that the conveying plunger (3) closes the conducting means (8) with respect to the second hollow space (7).

10. Device according to any one of the preceding claims, **characterised in that**
the dispensing opening has a stopper (12) arranged in it that closes the dispensing opening impermeable to the cement powder (5), in particular closes the dispensing opening permeable to gases, whereby the stopper (12) preferably is arranged in the dispensing opening such as to be mobile such that the stopper (12) is pushable out of the dispensing opening by pressing on the ready-mixed bone cement dough (62), whereby it is particularly preferred to have a marker means (16) that is visible from outside attached to the stopper (12).

11. Device according to any one of the preceding claims, **characterised in that**
the feedthrough (9) is closed by a stopper that is impermeable to gases and liquids, whereby the stopper is shiftable in the feedthrough (9) in the direction of the cartridge head (2), whereby the front side of the conveying plunger (3) preferably has a pin (10) arranged on it by means of which the stopper in the feedthrough (9) is pushable out of the feedthrough (9) and thus the feedthrough (9) is openable, whereby, particularly preferably, the pin (10) has an appropriate axial extension with respect to the cylindrical internal space of the cartridge (1) such that, upon the pin (10) and the stopper touching, the volume between the rear side of the dispensing plunger (4) and the front side of the conveying plunger (3) in the internal space of the cartridge (1) it is at least equal to the volume of the monomer liquid (6) in a monomer liquid container (32) that is arranged or is to be arranged in the device.

12. Method for the production of a bone cement dough (62), in particular a pasty polymethylmethacrylate bone cement dough, whereby the bone cement dough (62) is produced from a cement powder (5) and a monomer liquid (6) with a device according to any one of the preceding claims, **characterised by** the following steps proceeding in the order given:
A) conducting the monomer liquid (6) through the conducting means (8) into the second hollow space (7);
B) pushing the conveying plunger (3) in the direction of the dispensing plunger (4) until the dispensing plunger (3) closes all connections of the conducting means (8) to the second hollow space (7);
C) holding the device with the cartridge head (2) upwards and pushing the conveying plunger (3) further in the direction of the dispensing plunger (4), whereby air or gas is removed from the first hollow space (60) and from the second hollow space (7) through the cartridge head (2), and whereby the monomer liquid (6) is pushed from the second hollow space (7) into the cement powder (5) in the first hollow space (60);
D) the conveying plunger (3) pushing the dispensing plunger (4) in the direction of the dispensing opening, whereby the bone cement dough (62) produced in the first hollow space (60) flows out through the dispensing opening.

13. Method according to claim 12, **characterised in that**
the device is set up or held with the cartridge head (2) downwards in step A), and preferably in step B) as well, and, in step C), gas inclusions escape from the second hollow space (7) through the feedthrough (9), the first hollow space (60), and the dispensing opening in the cartridge head (2), preferably escape through a filter in the dispensing opening that is permeable to gases, but impermeable to the cement powder (5), whereby the filter closes the dispensing opening permeable for gases and impermeable for the cement powder (5).

14. Method according to claim 12 or 13, **characterised in that**
the conveying plunger (3) touches against the dispensing plunger (4) in step D) with matching surfaces and preferably the volume of the second hollow space (7) is reduced completely or down to a maximum of 1% in step C), and/or
the pressure acting on the bone cement dough (62) in step D) moves or pushes forward a stopper (12) in the dispensing opening, whereby the stopper (12) is preferably removed from the dispensing opening subsequently and, particularly preferably, then an application tube (64) is attached to the cartridge head (2) of the cartridge (1).

15. Method according to any one of the claims 12 to 14, **characterised in that** a monomer liquid container (32) containing the monomer liquid (6) is opened in a container (34) before step A) and the monomer liquid (6) is released in the container (34), whereby the container (34) is arranged on a rear side of the conveying plunger (3) that faces away from the dispensing plunger (4), and the monomer liquid (6) flows from the container (34) through the conducting means (8) into the second hollow space (7) in step A), whereby, preferably, the conveying plunger (3) in steps B) and C) and the conveying plunger (3) and the dispensing plunger (4) in step D) are driven by the container (34) being pushed or screwed into the cartridge (1).

## Revendications

1. Dispositif de fabrication d'une pâte de ciment osseux (62) à partir d'un liquide de monomère (6) et d'une poudre de ciment (5), servant de composants de départ de la pâte de ciment osseux (62), et d'évacuation de la pâte de ciment osseux (62), le dispositif présentant
une cartouche (1) avec un espace intérieur cylindrique,
une tête de cartouche (2) dotée d'un orifice d'évacuation pour l'expulsion de la pâte de ciment osseux (62), où la tête de cartouche (2) obture la cartouche (1) au niveau d'un côté avant de la cartouche (1) à l'exception de l'orifice d'évacuation,
un piston de transport (3), qui est disposé dans l'espace intérieur de la cartouche (1) et qui est logé dans l'espace intérieur de la cartouche (1) en pouvant être pressé en direction de l'orifice d'évacuation,
un piston d'évacuation (4), qui est disposé dans l'espace intérieur de la cartouche (1) entre l'orifice d'évacuation et le piston de transport (3) et qui est logé dans l'espace intérieur de la cartouche (1) en pouvant être pressé en direction de l'orifice d'évacuation, où une face arrière du piston d'évacuation (4) orientée vers le piston de transport (3) présente une partie creuse s'amenuisant en direction de la tête de cartouche (2),
une première cavité (60) qui est délimitée par la tête de cartouche (2), des parois intérieures de la cartouche (1) et par le piston d'évacuation (4), où la poudre de ciment (5) est disposée dans la première cavité (60),
une deuxième cavité (7) qui est une partie de l'espace intérieur cylindrique de la cartouche (1), où la deuxième cavité (7) est délimitée par le piston d'évacuation (4) et le piston de transport (3),
une partie coté arrière (11) de l'espace intérieur de la cartouche (1) dont le côté avant est délimité par un côté arrière du piston de transport (3) opposé au piston d'évacuation (4),
un élément de conduite (8), qui relie la deuxième cavité (7) avec la partie sur le côté arrière (11) de l'espace intérieur de la cartouche (1) passant devant le piston de transport (3) et qui étant perméable pour le liquide de monomère (6), où l'élément de conduite (8) peut être obturé par un déplacement du piston de transport (3) en direction du piston d'évacuation (4) par le piston de transport (3) contre la deuxième cavité (7), et
un passage (9), où le passage (9) commence au niveau d'une pointe de la partie creuse s'amenuisant, s'étend à travers le piston d'évacuation (4) et relie la première cavité (60) et la deuxième cavité (7) l'une avec l'autre en étant perméable pour le liquide de monomère (6) mais ne laissant pas passer la poudre de ciment (5).

2. Dispositif selon la revendication 1, **caractérisé en ce que**
un côté avant du piston de transport (3) orienté vers le piston d'évacuation (4) présente une surface proéminente en direction du piston d'évacuation (4) qui s'amenuise de préférence de manière continue en direction du piston d'évacuation (4).

3. Dispositif selon la revendication 1 ou la revendication 2, **caractérisé en ce**
**qu'**une partie creuse s'amenuisant en direction de la tête de cartouche (2) est une partie creuse s'amenuisant de manière continue sous la forme d'une surface en forme d'entonnoir (44) à partir de la paroi intérieure de la cartouche (1), où, de préférence, une surface proéminente sur le côté avant du piston de transport (3) peut être insérée dans la surface en forme d'entonnoir (44) sur le côté arrière du piston d'évacuation (4),où, de manière particulièrement préférée, le passage (9) dans le piston d'évacuation (4) débouche au point le plus bas de la surface en forme d'entonnoir (44) dans le piston d'évacuation (4).

4. Dispositif selon l'une des revendications précédentes, **caractérisé en ce**
**qu'**un côté avant du piston de transport (3) orienté vers le piston d'évacuation (4) et le côté arrière du piston d'évacuation (4) orienté vers le piston de transport (3) présentent des formes s'ajustant l'une à l'autre en suivant le même alignement, de sorte que le côté avant du piston de transport (3) est adjacent en surface au côté arrière du piston d'évacuation (4), lorsque le piston de transport (3) s'appuie contre le piston d'évacuation (4), où, de préférence, le volume de la deuxième cavité (7) peut être ainsi réduit jusqu'à au maximum 1 % du volume de la deuxième cavité (7) dans un état de départ.

5. Dispositif selon l'une des revendications précédentes, **caractérisé en ce que**
le côté arrière du piston d'évacuation (4) orienté vers le piston de transport (3) présente une surface en forme d'entonnoir (44) servant de partie creuse s'amenuisant, dans laquelle le passage (9) est disposé dans le point le plus bas, et un côté avant du piston de transport (3) orienté vers le piston d'évacuation (4) présente une surface en forme de cône proéminente (46) avec la même pente que la surface en forme d'entonnoir (44) sur le côté arrière du piston d'évacuation (4), où, de préférence, une pointe (10) cylindrique est disposée à la pointe de la surface en forme de cône proéminente (46) sur le coté avant du piston d'évacuation (4) qui peut s'insérer dans une partie creuse cylindrique sous forme d'une partie du passage (9) dans le piston d'évacuation (4).

6. Dispositif selon la revendication 5, **caractérisé en ce que**
la surface en forme d'entonnoir (44) sur le côté arrière du piston d'évacuation (4) présente un angle de pente d'au moins 45 °, de préférence, d'au moins 55 °, de manière particulièrement préférée d'au moins 60 ° ; et
la surface en forme de cône (46) sur le côté avant du piston de transport (3) présente un angle de pente lui correspondant d'au moins 45 °, de préférence, d'au moins 55 °, de manière particulièrement préférée d'au moins 60 °.

7. Dispositif selon l'une des revendications précédentes, **caractérisé en ce**
**qu'**un récipient (34) est disposé sur le côté arrière du piston de transport (3), dans lequel est disposé un récipient de liquide de monomère (32) contenant le liquide de monomère (6), notamment une ampoule en verre ou en matière plastique, où le récipient de liquide de monomère (32) peut être ouvert à l'intérieur du récipient (34) et où le récipient (34) est relié avec la partie (11) du côté arrière de l'espace intérieur de la cartouche (1) en laissant passer les liquides par le biais d'au moins une conduite de monomère (35), où, de préférence, un moyen d'ouverture (50) est disposé sur un côté du récipient (34) situé en face du piston de transport (3) avec lequel le récipient de liquide de monomère (32) peut être ouvert à l'intérieur du récipient (34), où, de manière particulièrement préférée le moyen d'ouverture (50) est un manchon (56) fixé sur un capuchon (54), où le capuchon (54) peut être vissé sur un filetage du récipient (34) et le capuchon (54) présente un filetage complémentaire à cette fin de sorte que, lors du vissage du capuchon (54) le récipient de liquide de monomère (32), notamment l'ampoule, est pressé avec le manchon (56) sur au moins une pointe proéminente (48) sur le côté intérieur du récipient (34) et ainsi le récipient de liquide de monomère (32), notamment l'ampoule, peut s'ouvrir en se cassant..

8. Dispositif selon la revendication 7, **caractérisé en ce que**
le récipient (34) présente un filetage extérieur qui peut se visser dans un filetage intérieur à une extrémité de la cartouche (1) située en face de la tête de cartouche (2), où, par le vissage du récipient (34) dans la cartouche (1), le piston de transport (3) peut être pressé en direction de l'orifice d'évacuation et le piston d'évacuation (4) peut être pressé en direction de l'orifice d'évacuation avec le piston de transport (3), où, de préférence, le filetage intérieur fait partie d'un manchon annulaire (36) qui est relié avec la cartouche (1) à l'extrémité de la cartouche (1) située en face de la tête de cartouche (2), et/ou
le côté extérieur du récipient (34) ne possède pas de filetage extérieur sur un premier segment partant d'un côté frontal orienté vers la tête de cartouche (2) et possède un filetage extérieur dans un deuxième segment.

9. Dispositif selon l'une des revendications précédentes, **caractérisé en ce que**
le moyen de conduite (8) s'arrête à un endroit sur la paroi intérieure de la cartouche (1) ou y débouche, qui est espacé axialement du piston d'évacuation (4) par rapport à l'axe de l'espace intérieur cylindrique de telle manière que le volume de la deuxième cavité (7) est au moins aussi grand que le volume du liquide de monomère (1) introduit par le moyen de conduite (8), notamment, du liquide de monomère (6) contenu dans le récipient de liquide de monomère lorsque le piston de transport (3) est déplacé d'une distance telle en direction du piston d'évacuation (4) que le piston de transport (3) obture le moyen de conduite (8) vis-à-vis de la deuxième cavité (7).

10. Dispositif selon l'une des revendications précédentes, **caractérisé en ce**
**qu'**un bouchon (12) est disposé dans l'orifice d'évacuation, qui ferme l'orifice d'évacuation pour la poudre de ciment (5) en ne la laissant pas passer, mais laisse en particulier passer les gaz, où, de préférence, le bouchon (12) est disposé mobile dans l'orifice d'évacuation de sorte que le bouchon (12) peut être pressé hors de l'orifice d'évacuation par une pression sur la pâte de ciment osseux (62) fraichement mélangée, où, de manière particulièrement préférée, un moyen de marquage (16) visible de l'extérieur est fixé sur le bouchon (12).

11. Dispositif selon l'une des revendications précédentes, **caractérisé en ce que**
le passage (9) est fermé par un bouchon ne laissant pas passer les gaz et les liquides, où le bouchon peut être déplacé dans le passage (9) en direction de la tête de cartouche (2), où, de préférence, une pointe (10) est disposée sur le côté avant du piston de transport (3), avec laquelle le bouchon peut être pressé hors du passage (9) dans le passage (9) et ainsi le passage (9) peut s'ouvrir, où, de manière particulièrement préférée, la pointe (10) possède une extension axiale telle par rapport à l'espace intérieur cylindrique de la cartouche (1) que, lors d'un effleurement de la pointe (10) avec le bouchon, le volume entre le côté arrière du piston d'évacuation (4) et le côté avant du piston de transport (3) dans l'espace intérieur de la cartouche (1) est au moins aussi grand que le volume du liquide de monomère (6) dans un récipient de liquide de monomère (32) qui est disposé ou peut être disposé dans le dispositif.

12. Procédé de fabrication d'une pâte de ciment osseux (62), notamment d'une pâte de ciment en poly méthyl méthacrylate en forme de pâte, dans lequel la pâte de ciment osseux (62) est fabriquée à partir d'une poudre de ciment (5) et d'un liquide de monomère (6) avec un dispositif selon l'une des revendications précédentes, **caractérisé par** les étapes suivantes se déroulant l'une après l'autre :
A) alimentation du liquide de monomère (6) par le moyen de conduite (8) dans la deuxième cavité (7),
B) exercice d'une pression sur le piston de transport (3) en direction du piston d'évacuation (4) jusqu'à ce que le piston de transport (3) ferme toutes les liaisons du moyen de conduite (8) vers la deuxième cavité (7),
C) maintien du dispositif avec la tête de cartouche (2) vers le haut et exercice d'une nouvelle pression sur le piston de transport (3) en direction du piston d'évacuation (4), où de l'air ou un gaz s'échappe de la première cavité (60) et de la deuxième cavité (7) par la tête de cartouche (2) et où le liquide de monomère (6) est pressé hors de la deuxième cavité (7) dans la poudre de ciment (5) dans la première cavité (60),
D) exercice d'une pression sur le piston d'évacuation (4) avec le piston de transport (3) en direction de l'orifice d'évacuation, où la pâte de ciment osseux (62) créée dans la première cavité (60) s'écoule par l'orifice d'évacuation.

13. Procédé selon la revendication 12, **caractérisé en ce que**
le dispositif est disposé ou maintenu avec la tête de cartouche (2) vers le bas dans l'étape A), et de préférence, également dans l'étape B), et, dans l'étape C), des inclusions de gaz s'échappent à partir de la deuxième cavité (7), à travers le passage (9), la première cavité (60) et l'orifice d'évacuation dans la tête de cartouche (2), de préférence, s'échappent par un filtre dans l'orifice d'évacuation laissant passer les gaz mais ne laissant pas passer la poudre de ciment (5), où le filtre obture l'orifice d'évacuation en laissant les gaz s'échapper et en ne laissant pas passer la poudre de ciment (5).

14. Procédé selon la revendication 12 ou la revendication 13, **caractérisé en ce que**
dans l'étape D), le piston de transport (3) est adjacent en surface avec le piston d'évacuation (4) et de préférence dans l'étape C), le volume de la deuxième cavité (7) est réduit totalement ou jusqu'à au maximum 1 %, et/ou
dans l'étape D, par la pression s'exerçant sur la pâte de ciment osseux (62), un bouchon (12) est déplacé ou pressé vers l'avant dans l'orifice d'évacuation, où, de préférence, le bouchon (12) est ensuite enlevé de l'orifice d'évacuation et de manière particulièrement préférée, un tube d'application (64) est ensuite fixé sur la tête de cartouche (2) de la cartouche (1).

15. Procédé selon l'une des revendications 12 à 14, **caractérisé en ce**
**qu'**avant l'étape A), un récipient de liquide de monomère (32) contenant le liquide de monomère (6) est ouvert dans un récipient (34), et le liquide de monomère (6) est libéré dans le récipient (34), où le récipient (34) est disposé, est notamment fixé, sur un côté arrière du piston de transport (3) opposé au piston d'évacuation (4), et le liquide de monomère (6) s'écoule dans l'étape A) hors du récipient (34) par le moyen de conduite (8) dans la deuxième cavité (7), où, de préférence, dans l'étape B) et dans l'étape C), le piston de transport (3), et dans l'étape D), le piston de transport (3) et le piston d'évacuation (4), sont entraînés par l'exercice d'une pression ou par un vissage du récipient (34) dans la cartouche (1).
